# EUROPEAN PATENT APPLICATION

(11) **EP 4 082 577 A1**
(43) Date of publication of application: **02.11.2022**
(21) Application number: 20907787.4
(22) Date of filing: 25.12.2020
(51) Int. Cl.: A61K 45/06, A61K 31/121, A61K 31/38, A61K 31/407, A61K 31/5415, A61K 31/542, A61K 31/63, A61K 35/74, A61K 39/04, A61K 39/39, A61K 39/395, A61K 41/00, A61N 5/10, A61P 11/00, A61P 35/00, A61P 37/04, A61P 43/00, G16H 20/00

(54) **CANCER TREATMENT METHOD AND MEDICINE**

(30) Priority: 27.12.2019 JP 2019238657; 09.10.2020 JP 2020171493
(71) Applicant: Zeria Pharmaceutical Co., Ltd., Tokyo 103-8351 (JP)
(72) Inventor: KOBAYASHI, Kunihiko, Saitama-shi, Saitama 330-0843 (JP); HORII, Takayuki, Tokyo 103-8351 (JP)
(74) Representative: Engelhard, Markus
(86) International application number: PCT/JP2020/048936
(87) International publication number: WO 2021/132636

(57) **Abstract**

The present disclosure provides a composition, a combination product, a medical device and the like for treating or preventing cancer or a tumor or preventing the recurrence of the cancer or the tumor. The present disclosure provides a composition, a combination product and a medical device for treating or preventing cancer or a tumor or preventing the recurrence of the cancer or the tumor, each of which comprises an immune checkpoint inhibitor and a dendritic cell direct activator or means. In another aspect, the present disclosure provides: a novel cancer treatment method which comprises carrying out a treatment of cancer by employing a combination of a treatment by the administration of an immune checkpoint inhibitor and a treatment for improving the sensitivity to the immune checkpoint inhibitor and, therefore, can be used as an immunotherapy that can be expected to have an excellent therapeutic effect; and a medicine which can be used for the cancer treatment method.

## Description

### [Technical Field]

The present disclosure relates to a method for treating cancer, a combination and a composition used for cancer treatment, and a program utilized for cancer treatment. The present disclosure particularly relates to a method, combination and composition, and program for cancer treatment based on immunotherapy. The present disclosure also relates to a novel technology for activating a dendritic cell.

### [Background Art]

Currently, examples of known cancer treatment that is commonly performed include surgical therapy (cancer tissue extraction by surgery), chemotherapy (administration of an anticancer agent comprising a molecularly targeted drug), radiation therapy (irradiation of radiation on cancer tissue), immunotherapy (enhancement of an immune function of a patient), and combinations thereof.

Among these therapies, immunotherapy is therapy that utilizes the ability of "immunity" that is innate in everyone to eliminate a foreign object invading the body, and enhances the immunity to try to treat cancer. While surgical therapy, chemotherapy, and radiation therapy treat cancer by using an external element (surgery/anticancer agent/radiation), immunotherapy treats cancer by mainly utilizing an individual's innate immunity (immune cells).

### [Summary of Invention]

### [Solution to Problem]

The inventors provide a novel technology for cancer treatment or prevention.

For example, the present disclosure provides the following as representative aspects.

### [Item 1]

A combination for use in treatment, prevention, or prevention of recurrence of cancer or tumor, comprising:
a) an immune checkpoint inhibitor; and
b) a direct dendritic cell activating agent or means,
characterized in that a) and b) are administered at different times or at the same time.

### [Item 2]

A composition or medical device for use in treatment, prevention, or prevention of recurrence of cancer or tumor of a subject, comprising a direct dendritic cell activating agent or means, characterized in that the composition or medical device is administered or used in combination with an immune checkpoint inhibitor.

### [Item 3]

A composition for use in treatment, prevention, or prevention of recurrence of cancer or tumor of a subject, comprising an immune checkpoint inhibitor, characterized in that the composition is administered in combination with a direct dendritic cell activating agent or means.

### [Item 4]

The combination, composition, or medical device of any of the preceding items, wherein the direct dendritic cell activating agent or means comprises at least one selected from the group consisting of a radiation therapy providing means and an immunoadjuvant.

### [Item 5]

The combination, composition, or medical device of any of the preceding items, wherein the immune checkpoint inhibitor comprises an inhibitor against at least one factor selected from the group consisting of PD-1, PD-L1, and CTLA-4.

### [Item 6]

The combination, composition, or medical device of any of the preceding items, wherein the immune checkpoint inhibitor comprises at least one agent selected from the group consisting of a PD-1 inhibitor, a PD-L1 inhibitor, and a CTLA-4 inhibitor.

### [Item 7]

The combination, composition, or medical device of any of the preceding items, wherein the cancer or tumor expresses an immune checkpoint factor.

### [Item 8]

The combination, composition, or medical device of any of the preceding items, wherein the cancer expresses PD-L1.

### [Item 9]

The combination, composition, or medical device of any of the preceding items, wherein the immune checkpoint inhibitor comprises at least one selected from the group consisting of nivolumab, pembrolizumab, and atezolizumab.

### [Item 10]

The combination, composition, or medical device of any of the preceding items, wherein the cancer is lung cancer.

### [Item 11]

The combination, composition, or medical device of any of the preceding items, wherein the cancer or tumor has an EGFR gene mutation.

### [Item 12]

The combination, composition, or medical device of any of the preceding items, wherein the treatment, prevention, or prevention of recurrence is performed on a subject who has received therapy with a tyrosine kinase inhibitor.

### [Item 13]

The combination, composition, or medical device of any of the preceding items, wherein the treatment, prevention, or prevention of recurrence is performed on a subject who has not received therapy with an immune checkpoint inhibitor.

### [Item 14]

The combination, composition, or medical device of any of the preceding items, wherein the treatment, prevention, or prevention of recurrence is performed on a subject for whom therapy with an immune checkpoint inhibitor was determined to be ineffective.

### [Item 15]

The combination, composition, or medical device of any of the preceding items, wherein the treatment, prevention, or prevention of recurrence is performed on a subject who received therapy with an immune checkpoint inhibitor which was effective, but subsequently experienced progressive disease (PD).

### [Item 16]

The combination, composition, or medical device of any of the preceding items, wherein the subject is CXCL10 positive in the subject's tumor.

### [Item 17]

The combination, composition, or medical device of any of the preceding items, wherein the subject is a CD8⁺ T cell^{low} subject.

### [Item 18]

A composition for use in directly activating a dendritic cell, comprising an immunoadjuvant.

### [Item 19]

The composition of any of the preceding items, wherein the immunoadjuvant comprises a human *Mycobacterium tuberculosis* hot water extract or a portion thereof.

### [Item 20]

The composition of any of the preceding items, wherein the activation is determined by measuring an increase in an amount of expression of CD80/86 on a surface of a dendritic cell in the presence of the immunoadjuvant compared to in the absence of the immunoadjuvant.

### [Item 21]

A combination for use in treatment, prevention, or prevention of recurrence of cancer or tumor, comprising:
a) an immune checkpoint inhibitor; and
b) an immunoadjuvant,
characterized in that a) and b) are administered at different times or at the same time.

### [Item 22]

A composition for use in treatment, prevention, or prevention of recurrence of cancer or tumor of a subject, comprising an immunoadjuvant, characterized in that the composition is administered in combination with an immune checkpoint inhibitor.

### [Item 23]

A composition for use in treatment, prevention, or prevention of recurrence of cancer or tumor of a subject, comprising an immune checkpoint inhibitor, characterized in that the composition is administered in combination with an immunoadjuvant.

### [Item 24]

The combination or composition of any of the preceding items, wherein the immunoadjuvant comprises a human *Mycobacterium tuberculosis* hot water extract or a portion thereof.

### [Item 25]

The composition of any of the preceding items, wherein the immune checkpoint inhibitor comprises an inhibitor against at least one factor selected from the group consisting of PD-1, PD-L1, and CTLA-4.

### [Item 26]

The combination or composition of any of the preceding items, wherein the immune checkpoint inhibitor comprises at least one agent selected from the group consisting of a PD-1 inhibitor, a PD-L1 inhibitor, and a CTLA-4 inhibitor.

### [Item 27]

A combination, or a composition or medical device for use in activating a dendritic cell, comprising the composition of any of the preceding items and a radiation therapy providing means.

### [Item 28]

The combination, composition, or medical device of any of the preceding items, wherein the radiation therapy providing means comprises at least one selected from the group consisting of a radiosensitizer, a radiation irradiating device, and a radioactive substance.

### [Item 29]

The combination, composition, or medical device of any of the preceding items, characterized in that the radiation therapy providing means is configured to provide palliative irradiation.

### [Item 30]

The combination, composition, or medical device of any of the preceding items, characterized in that the radiation therapy providing means is configured to irradiate to have an abscopal effect.

### [Item 31]

The combination, composition, or medical device of any of the preceding items, characterized in that the radiation therapy providing means is configured to irradiate a measurable lesion that is present other than a lesion that is a target.

### [Item 32]

The combination, composition, or medical device of any of the preceding items, further comprising a regulatory T cell inhibitor.

### [Item 33]

The combination, composition, or medical device of any of the preceding items, wherein the regulatory T cell inhibitor comprises a COX-2 inhibitor.

### [Item 34]

The combination, composition, or medical device of any of the preceding items, wherein the COX-2 inhibitor is selected from the group consisting of celecoxib, etodolac, meloxicam, nabumetone, zaltoprofen, and lornoxicam.

### [Item 35]

A composition or medical device for use in activating a dendritic cell, comprising a radiation therapy providing means.

### [Item 36]

The composition or medical device of any of the preceding items, wherein the radiation therapy providing means comprises at least one selected from the group consisting of a radiosensitizer, a radiation irradiating device, and a radioactive substance.

### [Item 37]

The composition or medical device of any of the preceding items, wherein the activation is determined by measuring an increase in an amount of expression of CD80/86 on a surface of a dendritic cell in the presence of the immunoadjuvant compared to in the absence of the immunoadjuvant.

### [Item 38]

The composition or medical device of any of the preceding items, characterized in that the radiation therapy providing means is configured to provide palliative irradiation.

### [Item 39]

The composition or medical device of any of the preceding items, characterized in that the radiation therapy providing means is configured to irradiate to have an abscopal effect.

### [Item 40]

The composition or medical device of any of the preceding items, characterized in that the radiation therapy providing means is configured to irradiate a measurable lesion that is present other than a lesion that is a target.

### [Item 41]

A program utilized for cancer treatment or prevention of a patient, characterized by causing a computer to execute:
a step of performing either one or both of first therapy with an immune checkpoint inhibitor and/or a dendritic cell activating agent or means on the patient; and
a step of identifying an optimal therapy program based on a reaction of the patient.

### [Item 42]

The program of any of the preceding items, characterized in that the reaction of the patient is a predetermined change in a medical image of the patient and a measurement value of a tumor marker in the patient.

### [Item 43]

A medicament for use in prevention or treatment of cancer in a patient,
the medicament comprising a combination of an immune checkpoint and a dendritic cell activating agent or means, characterized in that dosage and administration are identified based on information on an optimization identifying marker of the combination obtained from the patient, and a therapeutically or prophylactically effective combination of the immune checkpoint and the dendritic cell activating agent or means is administered based on the identified dosage and administration.

### [Item 44]

A medicament or device for use in treatment, prevention, or prevention of recurrence of cancer, cancer, or tumor of a subject with a specific regimen,
the medicament comprising a combination of an immune checkpoint and a dendritic cell activating agent or means, wherein the regimen comprises:
a) a step of administering an immune checkpoint inhibitor; and
b) a step of administering a dendritic cell activating agent.

### [Item 45]

The medicament or device of any of the preceding items, wherein the subject is a subject who has not received therapy with an immune checkpoint inhibitor or a subject for whom therapy with an immune checkpoint inhibitor alone is ineffective.

### [Item 46]

The medicament or device of any of the preceding items, wherein, in the specific regimen, the immune checkpoint inhibitor:
i) is nivolumab and administered at 240 mg every 2 weeks;
ii) is pembrolizumab and administered at 200 mg at an interval of 3 weeks or at 400 mg at an interval of 6 weeks; or
iii) is atezolizumab and administered at 1200 mg at an interval of 3 weeks.

### [Item 47]

The medicament or device of any of the preceding items, wherein the specific regimen further comprises:
c) a step of performing radiation therapy.

### [Item 48]

The medicament or device of any of the preceding items, characterized in that the radiation therapy is palliative irradiation other than brain metastasis, and irradiation is performed 10 fractions at 3 Gy or 20 to 25 fractions at 2 Gy.

### [Item 49]

The medicament or device of any of the preceding items, wherein the dendritic cell activating agent or means comprises Extract Z that is administered by aspirating a liquid medicine into a 1 mL syringe and subcutaneously injecting 0.05 mL thereof twice a week.

### [Item A1]

A method for treatment, prevention, or prevention of recurrence of cancer or tumor in a subject, comprising administering:
a) an effective amount of an immune checkpoint inhibitor; and
b) an effective amount of a direct dendritic cell activating agent or means
   to the subject at different times or at the same time.

### [Item A2]

A method for treatment, prevention, or prevention of recurrence of cancer or tumor in a subject, comprising administering or using an effective amount of a direct dendritic cell activating agent or means to the subject, wherein administration of the direct dendritic cell activating agent or means comprises administration or use in combination with an effective amount of an immune checkpoint inhibitor.

### [Item A3]

A method for treatment, prevention, or prevention of recurrence of cancer or tumor of a subject, comprising administering an effective amount of an immune checkpoint inhibitor to the subject, wherein administration of the immune checkpoint inhibitor comprises administration or use in combination with an effective amount of a direct dendritic cell activating agent or means.

### [Item A4]

The method of any of the preceding items, wherein the direct dendritic cell activating agent or means comprises at least one selected from the group consisting of a radiation therapy providing means and an immunoadjuvant.

### [Item A5]

The method of any of the preceding items, wherein the immune checkpoint inhibitor comprises an inhibitor against at least one factor selected from the group consisting of PD-1, PD-L1, and CTLA-4.

### [Item A6]

The method of any of the preceding items, wherein the immune checkpoint inhibitor comprises at least one agent selected from the group consisting of a PD-1 inhibitor, a PD-L1 inhibitor, and a CTLA-4 inhibitor.

### [Item A7]

The method of any of the preceding items, wherein the cancer or tumor expresses an immune checkpoint factor.

### [Item A8]

The method of any of the preceding items, wherein the cancer expresses PD-L1.

### [Item A9]

The method of any of the preceding items, wherein the immune checkpoint inhibitor comprises at least one selected from the group consisting of nivolumab, pembrolizumab, and atezolizumab.

### [Item A10]

The method of any of the preceding items, wherein the cancer is lung cancer.

### [Item A11]

The method of any of the preceding items, wherein the cancer or tumor has an EGFR gene mutation.

### [Item A12]

The method of any of the preceding items, wherein the method is performed on a subject who has received therapy with a tyrosine kinase inhibitor.

### [Item A13]

The method of any of the preceding items, wherein the method is performed on a subject who has not received therapy with an immune checkpoint inhibitor.

### [Item A14]

The method of any of the preceding items, wherein the method is performed on a subject for whom therapy with an immune checkpoint inhibitor was determined to be ineffective.

### [Item A15]

The method of any of the preceding items, wherein the method is performed on a subject who received therapy with an immune checkpoint inhibitor which was effective, but subsequently experienced progressive disease (PD).

### [Item A16]

The method of any of the preceding items, wherein the subject is CXCL10 positive in the subject's tumor.

### [Item A17]

The method of any of the preceding items, wherein the subject is a CD8⁺ T cell^{low} subject.

### [Item A18]

A method for directly activating a dendritic cell in a subject, comprising administering an effective amount of an immunoadjuvant to the subject.

### [Item A19]

The method of any of the preceding items, wherein the immunoadjuvant comprises a human *Mycobacterium tuberculosis* hot water extract or a portion thereof.

### [Item A20]

The method of any of the preceding items, wherein the activation is determined by measuring an increase in an amount of expression of CD80/86 on a surface of a dendritic cell in the presence of the immunoadjuvant compared to in the absence of the immunoadjuvant.

### [Item A21]

A method for treatment, prevention, or prevention of recurrence of cancer or tumor in a subject, comprising administering:
a) an effective amount of an immune checkpoint inhibitor; and
b) an effective amount of an immunoadjuvant
   to the subject at different times or at the same time.

### [Item A22]

A method for treatment, prevention, or prevention of recurrence of cancer or tumor in a subject, comprising administering an effective amount of an immunoadjuvant to the subject, wherein administration of the immunoadjuvant comprises administration in combination with an effective amount of an immune checkpoint inhibitor.

### [Item A23]

A method for treatment, prevention, or prevention of recurrence of cancer or tumor of a subject, comprising administering an immune checkpoint inhibitor to the subject, wherein the immune checkpoint inhibitor is administered in combination with an immunoadjuvant.

### [Item A24]

The method of any of the preceding items, wherein the immunoadjuvant comprises a human *Mycobacterium tuberculosis* hot water extract or a portion thereof.

### [Item A25]

The method of any of the preceding items, wherein the immune checkpoint inhibitor comprises an inhibitor against at least one factor selected from the group consisting of PD-1, PD-L1, and CTLA-4.

### [Item A26]

The method of any of the preceding items, wherein the immune checkpoint inhibitor comprises at least one agent selected from the group consisting of a PD-1 inhibitor, a PD-L1 inhibitor, and a CTLA-4 inhibitor.

### [Item A27]

A method for activating a dendritic cell, wherein a radiation therapy providing means is further used.

### [Item A28]

The method of any of the preceding items, wherein the radiation therapy providing means comprises at least one selected from the group consisting of a radiosensitizer, a radiation irradiating device, and a radioactive substance.

### [Item A29]

The method of any of the preceding items, wherein the radiation therapy providing means is configured to provide palliative irradiation.

### [Item A30]

The method of any of the preceding items, wherein the radiation therapy providing means is configured to irradiate to have an abscopal effect.

### [Item A31]

The method of any of the preceding items, wherein the radiation therapy providing means is configured to irradiate a measurable lesion that is present other than a lesion that is a target.

### [Item A32]

The method of any of the preceding items, wherein a regulatory T cell inhibitor is further administered.

### [Item A33]

The method of any of the preceding items, wherein the regulatory T cell inhibitor comprises a COX-2 inhibitor.

### [Item A34]

The method of any of the preceding items, wherein the COX-2 inhibitor is selected from the group consisting of celecoxib, etodolac, meloxicam, nabumetone, zaltoprofen, and lornoxicam.

### [Item A35]

A method for activating a dendritic cell, comprising using a radiation therapy providing means for a subject.

### [Item A36]

The method of any of the preceding items, wherein the radiation therapy providing means comprises at least one selected from the group consisting of a radiosensitizer, a radiation irradiating device, and a radioactive substance.

### [Item A37]

The method of any of the preceding items, wherein the activation is determined by measuring an increase in an amount of expression of CD80/86 on a surface of a dendritic cell in the presence of the immunoadjuvant compared to in the absence of the immunoadjuvant.

### [Item A38]

The method of any of the preceding items, wherein the radiation therapy providing means is configured to provide palliative irradiation.

### [Item A39]

The method of any of the preceding items, wherein the radiation therapy providing means is configured to irradiate to have an abscopal effect.

### [Item A40]

The method of any of the preceding items, wherein the radiation therapy providing means is configured to irradiate a measurable lesion that is present other than a lesion that is a target.

### [Item A41]

A method for treatment or prevention of cancer of a patient, comprising causing a computer to execute:
a step of performing either one or both of first therapy with an effective amount of an immune checkpoint inhibitor and/or an effective amount of a dendritic cell activating agent or means on the patient; and
a step of identifying an optimal therapy program based on a reaction of the patient.

### [Item A42]

The method of any of the preceding items, characterized in that the reaction of the patient is a predetermined change in a medical image of the patient and a measurement value of a tumor marker in the patient.

### [Item A43]

A method for prevention or treatment of cancer in a patient, comprising administering a combination of an effective amount of an immune checkpoint and an effective amount of a dendritic cell activating agent or means,
wherein, based on information on an optimization identifying marker of the combination obtained from the patient, dosage and administration of a therapeutically or prophylactically effective combination of the immune checkpoint and the dendritic cell activating agent or means are identified, and the combination is administered based on the identified dosage and administration.

### [Item A44]

A method for treatment, prevention, or prevention of recurrence of cancer or tumor of a subject with a specific regimen,
the method comprising administering a combination of an effective amount of an immune checkpoint and an effective amount of a dendritic cell activating agent or means, wherein the regimen comprises:
a) a step of administering an effective amount of an immune checkpoint inhibitor; and
b) a step of administering an effective amount of a dendritic cell activating agent.

### [Item A45]

The method of any of the preceding items, wherein the subject is a subject who has not received therapy with an immune checkpoint inhibitor or a subject for whom therapy with an immune checkpoint inhibitor alone is ineffective.

### [Item A46]

The method of any of the preceding items, wherein, in the specific regimen, the immune checkpoint inhibitor:
i) is nivolumab and administered at 240 mg every 2 weeks;
ii) is pembrolizumab and administered at 200 mg at an interval of 3 weeks or at 400 mg at an interval of 6 weeks; or
iii) is atezolizumab and administered at 1200 mg at an interval of 3 weeks.

### [Item A47]

The method of any of the preceding items, wherein the specific regimen further comprises:
c) a step of performing radiation therapy.

### [Item A48]

The method of any of the preceding items, wherein the radiation therapy is palliative irradiation other than brain metastasis, and irradiation is performed 10 fractions at 3 Gy or 20 to 25 fractions at 2 Gy.

### [Item A49]

The method of any of the preceding items, wherein the dendritic cell activating agent or means comprises Extract Z that is administered by aspirating a liquid medicine into a 1 mL syringe and subcutaneously injecting 0.05 mL thereof twice a week.

### [Item B1]

Use of a) an immune checkpoint inhibitor and b) a direct dendritic cell activating agent or means in the manufacture of a medicament for therapy, prevention, or prevention of recurrence of cancer or tumor.

### [Item B2]

Use of a direct dendritic cell activating agent or means in the manufacture of a medicament that is used in combination with an immune checkpoint inhibitor for therapy, prevention, or prevention of recurrence of cancer or tumor of a subject.

### [Item B3]

Use of an immune checkpoint inhibitor in the manufacture of a medicament that is used in combination with a direct dendritic cell activating agent or means for treatment, prevention, or prevention of recurrence of cancer or tumor of a subject.

### [Item B4]

The use of any of the preceding items, wherein the direct dendritic cell activating agent or means comprises at least one selected from the group consisting of a radiation therapy providing means and an immunoadjuvant.

### [Item B5]

The use of any of the preceding items, wherein the immune checkpoint inhibitor comprises an inhibitor against at least one factor selected from the group consisting of PD-1, PD-L1, and CTLA-4.

### [Item B6]

The use of any of the preceding items, wherein the immune checkpoint inhibitor comprises at least one agent selected from the group consisting of a PD-1 inhibitor, a PD-L1 inhibitor, and a CTLA-4 inhibitor.

### [Item B7]

The use of any of the preceding items, wherein the cancer or tumor expresses an immune checkpoint factor.

### [Item B8]

The use of any of the preceding items, wherein the cancer expresses PD-L1.

### [Item B9]

The use of any of the preceding items, wherein the immune checkpoint inhibitor comprises at least one selected from the group consisting of nivolumab, pembrolizumab, and atezolizumab.

### [Item B10]

The use of any of the preceding items, wherein the cancer is lung cancer.

### [Item B11]

The use of any of the preceding items, wherein the cancer or tumor has an EGFR gene mutation.

### [Item B12]

The use of any of the preceding items, wherein the treatment, prevention, or prevention of recurrence is performed on a subject who has received therapy with a tyrosine kinase inhibitor.

### [Item B13]

The use of any of the preceding items, wherein the treatment, prevention, or prevention of recurrence is performed on a subject who has not received therapy with an immune checkpoint inhibitor.

### [Item B14]

The use of any of the preceding items, wherein the treatment, prevention, or prevention of recurrence is performed on a subject for whom therapy with an immune checkpoint inhibitor was determined to be ineffective.

### [Item B15]

The use of any of the preceding items, wherein the treatment, prevention, or prevention of recurrence is performed on a subject who received therapy with an immune checkpoint inhibitor which was effective, but subsequently experienced progressive disease (PD).

### [Item B16]

The use of any of the preceding items, wherein the subject is CXCL10 positive in the subject's tumor.

### [Item B17]

The use of any of the preceding items, wherein the subject is a CD8⁺ T cell^{low} subject.

### [Item B18]

Use of an immunoadjuvant in the manufacture of a medicament for directly activating a dendritic cell.

### [Item B19]

The use of any of the preceding items, wherein the immunoadjuvant comprises a human *Mycobacterium tuberculosis* hot water extract or a portion thereof.

### [Item B20]

The use of any of the preceding items, wherein the activation is determined by measuring an increase in an amount of expression of CD80/86 on a surface of a dendritic cell in the presence of the immunoadjuvant compared to in the absence of the immunoadjuvant.

### [Item B21]

Use of a) an immune checkpoint inhibitor and b) an immunoadjuvant in the manufacture of a medicament for treatment, prevention, or prevention of recurrence of cancer or tumor.

### [Item B22]

Use of an immunoadjuvant in the manufacture of a medicament that is used in combination with an immune checkpoint inhibitor for treatment, prevention, or prevention of recurrence of cancer or tumor.

### [Item B23]

Use of an immune checkpoint inhibitor in the manufacture of a medicament that is used in combination with an immunoadjuvant for treatment, prevention, or prevention of recurrence of cancer or tumor.

### [Item B24]

The use of any of the preceding items, wherein the immunoadjuvant comprises a human *Mycobacterium tuberculosis* hot water extract or a portion thereof.

### [Item B25]

The use of any of the preceding items, wherein the immune checkpoint inhibitor comprises an inhibitor against at least one factor selected from the group consisting of PD-1, PD-L1, and CTLA-4.

### [Item B26]

The use of any of the preceding items, wherein the immune checkpoint inhibitor comprises at least one agent selected from the group consisting of a PD-1 inhibitor, a PD-L1 inhibitor, and a CTLA-4 inhibitor.

### [Item B27]

The use of any of the preceding items, characterized in that a radiation therapy providing means is further used for the subject.

### [Item B28]

The use of any of the preceding items, wherein the radiation therapy providing means comprises at least one selected from the group consisting of a radiosensitizer, a radiation irradiating device, and a radioactive substance.

### [Item B29]

The use of any of the preceding items, characterized in that the radiation therapy providing means is configured to provide palliative irradiation.

### [Item B30]

The use of any of the preceding items, characterized in that the radiation therapy providing means is configured to irradiate to have an abscopal effect.

### [Item B31]

The use of any of the preceding items, characterized in that the radiation therapy providing means is configured to irradiate a measurable lesion that is present other than a lesion that is a target.

### [Item B32]

The use of any of the preceding items, characterized in that a regulatory T cell inhibitor is further administered to the subject.

### [Item B33]

The use of any of the preceding items, wherein the regulatory T cell inhibitor comprises a COX-2 inhibitor.

### [Item B34]

The use of any of the preceding items, wherein the COX-2 inhibitor is selected from the group consisting of celecoxib, etodolac, meloxicam, nabumetone, zaltoprofen, and lornoxicam.

### [Item B35]

Use of a radiation therapy providing means in the manufacture of a medicament for activating a dendritic cell.

### [Item B36]

The use of any of the preceding items, wherein the radiation therapy providing means comprises at least one selected from the group consisting of a radiosensitizer, a radiation irradiating device, and a radioactive substance.

### [Item B37]

The use of any of the preceding items, wherein the activation is determined by measuring an increase in an amount of expression of CD80/86 on a surface of a dendritic cell in the presence of the immunoadjuvant compared to in the absence of the immunoadjuvant.

### [Item B38]

The use of any of the preceding items, characterized in that the radiation therapy providing means is configured to provide palliative irradiation.

### [Item B39]

The use of any of the preceding items, characterized in that the radiation therapy providing means is configured to irradiate to have an abscopal effect.

### [Item B40]

The use of any of the preceding items, characterized in that the radiation therapy providing means is configured to irradiate a measurable lesion that is present other than a lesion that is a target.

### [Item B41]

A program utilized for cancer treatment or prevention of a patient, characterized by causing a computer to execute:
a step of performing either one or both of first therapy with an immune checkpoint inhibitor and/or a dendritic cell activating agent or means on the patient; and
a step of identifying an optimal therapy program based on a reaction of the patient.

### [Item B42]

The program of any of the preceding items, characterized in that the reaction of the patient is a predetermined change in a medical image of the patient and a measurement value of a tumor marker in the patient.

### [Item B43]

Use of a combination of an immune checkpoint and a dendritic cell activating agent or means in the manufacture of a medicament for prevention or treatment of cancer in a patient,
wherein the medicament is characterized in that dosage and administration are identified based on information on an optimization identifying marker of the combination obtained from the patient, and a therapeutically or prophylactically effective combination of the immune checkpoint and the dendritic cell activating agent or means is administered based on the identified dosage and administration.

### [Item B44]

Use of an immune checkpoint inhibitor and a dendritic cell activating agent in the manufacture of a medicament for treatment, prevention, or prevention of recurrence of cancer or tumor of a subject with a specific regimen.

### [Item B45]

The use of any of the preceding items, wherein the subject is a subject who has not received therapy with an immune checkpoint inhibitor or a subject for whom therapy with an immune checkpoint inhibitor alone is ineffective.

### [Item B46]

The use of any of the preceding items, wherein, in the specific regimen, the immune checkpoint inhibitor:
i) is nivolumab and administered at 240 mg every 2 weeks;
ii) is pembrolizumab and administered at 200 mg at an interval of 3 weeks or at 400 mg at an interval of 6 weeks; or
iii) is atezolizumab and administered at 1200 mg at an interval of 3 weeks.

### [Item B47]

The use of any of the preceding items, wherein the specific regimen further comprises:
c) a step of performing radiation therapy.

### [Item B48]

The use of any of the preceding items, characterized in that the radiation therapy is palliative irradiation other than brain metastasis, and irradiation is performed 10 fractions at 3 Gy or 20 to 25 fractions at 2 Gy.

### [Item B49]

The use of any of the preceding items, wherein the dendritic cell activating agent or means comprises Extract Z that is administered by aspirating a liquid medicine into a 1 mL syringe and subcutaneously injecting 0.05 mL thereof twice a week.

### [Item C1]

An immune checkpoint inhibitor for use in treatment, prevention, or prevention of recurrence of cancer or tumor, characterized by being used in combination with a direct dendritic cell activating agent or means.

### [Item C2]

A direct dendritic cell activating agent or means for use in treatment, prevention, or prevention of recurrence of cancer or tumor of a subject, characterized by being administered in combination with an immune checkpoint inhibitor.

### [Item C3]

An immune checkpoint inhibitor for use in treatment, prevention, or prevention of recurrence of cancer or tumor of a subject, characterized by being administered in combination with a direct dendritic cell activating agent or means.

### [Item C4]

The immune checkpoint inhibitor or direct dendritic cell activating agent or means of any of the preceding items, wherein the direct dendritic cell activating agent or means comprises at least one selected from the group consisting of a radiation therapy providing means and an immunoadjuvant.

### [Item C5]

The immune checkpoint inhibitor or direct dendritic cell activating agent or means of any of the preceding items, wherein the immune checkpoint inhibitor comprises an inhibitor against at least one factor selected from the group consisting of PD-1, PD-L1, and CTLA-4.

### [Item C6]

The immune checkpoint inhibitor or direct dendritic cell activating agent or means of any of the preceding items, wherein the immune checkpoint inhibitor comprises at least one agent selected from the group consisting of a PD-1 inhibitor, a PD-L1 inhibitor, and a CTLA-4 inhibitor.

### [Item C7]

The immune checkpoint inhibitor or direct dendritic cell activating agent or means of any of the preceding items, wherein the cancer or tumor expresses an immune checkpoint factor.

### [Item C8]

The immune checkpoint inhibitor or direct dendritic cell activating agent or means of any of the preceding items, wherein the cancer expresses PD-L1.

### [Item C9]

The immune checkpoint inhibitor or direct dendritic cell activating agent or means of any of the preceding items, wherein the immune checkpoint inhibitor comprises at least one selected from the group consisting of nivolumab, pembrolizumab, and atezolizumab.

### [Item C10]

The immune checkpoint inhibitor or direct dendritic cell activating agent or means of any of the preceding items, wherein the cancer is lung cancer.

### [Item C11]

The immune checkpoint inhibitor or direct dendritic cell activating agent or means of any of the preceding items, wherein the cancer or tumor has an EGFR gene mutation.

### [Item C12]

The immune checkpoint inhibitor or direct dendritic cell activating agent or means of any of the preceding items, wherein the treatment, prevention, or prevention of recurrence is performed on a subject who has received therapy with a tyrosine kinase inhibitor.

### [Item C13]

The immune checkpoint inhibitor or direct dendritic cell activating agent or means of any of the preceding items, wherein the treatment, prevention, or prevention of recurrence is performed on a subject who has not received therapy with an immune checkpoint inhibitor.

### [Item C14]

The immune checkpoint inhibitor or direct dendritic cell activating agent or means of any of the preceding items, wherein the treatment, prevention, or prevention of recurrence is performed on a subject for whom therapy with an immune checkpoint inhibitor was determined to be ineffective.

### [Item C15]

The immune checkpoint inhibitor or direct dendritic cell activating agent or means of any of the preceding items, wherein the treatment, prevention, or prevention of recurrence is performed on a subject who received therapy with an immune checkpoint inhibitor which was effective, but subsequently experienced progressive disease (PD).

### [Item C16]

The immune checkpoint inhibitor or direct dendritic cell activating agent or means of any of the preceding items, wherein the subject is CXCL10 positive in the subject's tumor.

### [Item C17]

The immune checkpoint inhibitor or direct dendritic cell activating agent or means of any of the preceding items, wherein the subject is a CD8⁺ T cell^{low} subject.

### [Item C18]

An immunoadjuvant for use in directly activating a dendritic cell.

### [Item C19]

The immunoadjuvant of any of the preceding items, comprising a human *Mycobacterium tuberculosis* hot water extract or a portion thereof.

### [Item C20]

The immunoadjuvant of any of the preceding items, wherein the activation is determined by measuring an increase in an amount of expression of CD80/86 on a surface of a dendritic cell in the presence of the immunoadjuvant compared to in the absence of the immunoadjuvant.

### [Item C21]

An immune checkpoint inhibitor for use in treatment, prevention, or prevention of recurrence of cancer or tumor, characterized by being administered in combination with an immunoadjuvant.

### [Item C22]

An immunoadjuvant for use in treatment, prevention, or prevention of recurrence of cancer or tumor of a subject, characterized by being administered in combination with an immune checkpoint inhibitor.

### [Item C23]

An immune checkpoint inhibitor for use in treatment, prevention, or prevention of recurrence of cancer or tumor of a subject, characterized by being administered in combination with an immunoadjuvant.

### [Item C24]

The immunoadjuvant or immune checkpoint inhibitor of any of the preceding items, wherein the immunoadjuvant comprises a human *Mycobacterium tuberculosis* hot water extract or a portion thereof.

### [Item C25]

The immunoadjuvant or immune checkpoint inhibitor of any of the preceding items, wherein the immune checkpoint inhibitor comprises an inhibitor against at least one factor selected from the group consisting of PD-1, PD-L1, and CTLA-4.

### [Item C26]

The immunoadjuvant or immune checkpoint inhibitor of any of the preceding items, wherein the immune checkpoint inhibitor comprises at least one agent selected from the group consisting of a PD-1 inhibitor, a PD-L1 inhibitor, and a CTLA-4 inhibitor.

### [Item C27]

The immunoadjuvant or immune checkpoint inhibitor of any of the preceding items, characterized in that a radiation therapy providing means is further administered.

### [Item C28]

The immunoadjuvant or immune checkpoint inhibitor of any of the preceding items, wherein the radiation therapy providing means comprises at least one selected from the group consisting of a radiosensitizer, a radiation irradiating device, and a radioactive substance.

### [Item C29]

The immunoadjuvant or immune checkpoint inhibitor of any of the preceding items, characterized in that the radiation therapy providing means is configured to provide palliative irradiation.

### [Item C30]

The immunoadjuvant or immune checkpoint inhibitor of any of the preceding items, characterized in that the radiation therapy providing means is configured to irradiate to have an abscopal effect.

### [Item C31]

The immunoadjuvant or immune checkpoint inhibitor of any of the preceding items, characterized in that the radiation therapy providing means is configured to irradiate a measurable lesion that is present other than a lesion that is a target.

### [Item C32]

The immunoadjuvant or immune checkpoint inhibitor of any of the preceding items, further comprising a regulatory T cell inhibitor.

### [Item C33]

The immunoadjuvant or immune checkpoint inhibitor of any of the preceding items, wherein the regulatory T cell inhibitor comprises a COX-2 inhibitor.

### [Item C34]

The immunoadjuvant or immune checkpoint inhibitor of any of the preceding items, wherein the COX-2 inhibitor is selected from the group consisting of celecoxib, etodolac, meloxicam, nabumetone, zaltoprofen, and lornoxicam.

### [Item C35]

A radiation therapy providing means for use in activating a dendritic cell.

### [Item C36]

The radiation therapy providing means of any of the preceding items, comprising at least one selected from the group consisting of a radiosensitizer, a radiation irradiating device, and a radioactive substance.

### [Item C37]

The radiation therapy providing means of any of the preceding items, wherein the activation is determined by measuring an increase in an amount of expression of CD80/86 on a surface of a dendritic cell in the presence of the immunoadjuvant compared to in the absence of the immunoadjuvant.

### [Item C38]

The radiation therapy providing means of any of the preceding items, characterized by being configured to provide palliative irradiation.

### [Item C39]

The radiation therapy providing means of any of the preceding items, characterized by being configured to irradiate to have an abscopal effect.

### [Item C40]

The radiation therapy providing means of any of the preceding items, characterized by being configured to irradiate a measurable lesion that is present other than a lesion that is a target.

### [Item C41]

A program utilized for cancer treatment or prevention of a patient, characterized by causing a computer to execute:
a step of performing either one or both of first therapy with an immune checkpoint inhibitor and/or a dendritic cell activating agent or means on the patient; and
a step of identifying an optimal therapy program based on a reaction of the patient.

### [Item C42]

The program of any of the preceding items, characterized in that the reaction of the patient is a predetermined change in a medical image of the patient and a measurement value of a tumor marker in the patient.

### [Item C43]

An immune checkpoint inhibitor for prevention or treatment of cancer in a patient, the immune checkpoint inhibitor characterized by being administered in combination with a dendritic cell activating agent or means, characterized in that dosage and administration are identified based on information on an optimization identifying marker of the combination obtained from the patient, and a therapeutically or prophylactically effective combination of the immune checkpoint and the dendritic cell activating agent or means is administered based on the identified dosage and administration.

### [Item C43A]

A dendritic cell activating agent or means for prevention or treatment of cancer in a patient, the dendritic cell activating agent or means characterized by being administered in combination with an immune checkpoint inhibitor, characterized in that dosage and administration are identified based on information on an optimization identifying marker of the combination obtained from the patient, and a therapeutically or prophylactically effective combination of the immune checkpoint and the dendritic cell activating agent or means is administered based on the identified dosage and administration.

### [Item C44]

An immune checkpoint inhibitor for use in treatment, prevention, or prevention of recurrence of cancer, cancer, or tumor of a subject with a specific regimen, wherein the regimen comprises a step of administering the immune checkpoint inhibitor in combination with a dendritic cell activating agent.

### [Item C44A]

A dendritic cell activating agent for use in treatment, prevention, or prevention of recurrence of cancer, cancer, or tumor of a subject with a specific regimen, wherein the regimen comprises a step of administering the dendritic cell activating agent in combination with an immune checkpoint inhibitor.

### [Item C45]

The immune checkpoint inhibitor or dendritic cell activating agent of any of the preceding items, wherein the subject is a subject who has not received therapy with an immune checkpoint inhibitor or a subject for whom therapy with an immune checkpoint inhibitor alone is ineffective.

### [Item C46]

The immune checkpoint inhibitor or dendritic cell activating agent of any of the preceding items, wherein, in the specific regimen, the immune checkpoint inhibitor:
i) is nivolumab and administered at 240 mg every 2 weeks;
ii) is pembrolizumab and administered at 200 mg at an interval of 3 weeks or at 400 mg at an interval of 6 weeks; or
iii) is atezolizumab and administered at 1200 mg at an interval of 3 weeks.

### [Item C47]

The immune checkpoint inhibitor or dendritic cell activating agent of any of the preceding items, wherein the specific regimen further comprises:
c) a step of performing radiation therapy.

### [Item C48]

The immune checkpoint inhibitor or dendritic cell activating agent of any of the preceding items, characterized in that the radiation therapy is palliative irradiation other than brain metastasis, and irradiation is performed 10 fractions at 3 Gy or 20 to 25 fractions at 2 Gy.

### [Item C49]

The immune checkpoint inhibitor or dendritic cell activating agent of any of the preceding items, wherein the dendritic cell activating agent or means comprises Extract Z that is administered by aspirating a liquid medicine into a 1 mL syringe and subcutaneously injecting 0.05 mL thereof twice a week.

The present disclosure also provides the following.

### [Item X1]

A combination for use in treatment, prevention, or prevention of recurrence of cancer or tumor, comprising:
a) an immune checkpoint inhibitor; and
b) a direct dendritic cell activating agent or means,
characterized in that a) and b) are administered at different times or at the same time.

### [Item X2]

A composition or medical device for use in treatment, prevention, or prevention of recurrence of cancer or tumor of a subject, comprising a direct dendritic cell activating agent or means, characterized in that the composition or medical device is administered or used in combination with an immune checkpoint inhibitor.

### [Item X3]

A composition for use in treatment, prevention, or prevention of recurrence of cancer or tumor of a subject, comprising an immune checkpoint inhibitor, characterized in that the composition is administered in combination with a direct dendritic cell activating agent or means.

### [Item X4]

The combination, composition, or medical device of any of the preceding items, wherein the direct dendritic cell activating agent or means comprises at least one selected from the group consisting of a radiation therapy providing means and an immunoadjuvant.

### [Item X5]

The combination, composition, or medical device of any of the preceding items, wherein the direct dendritic cell activating agent or means comprises a radiation therapy providing means and an immunoadjuvant.

### [Item X6]

The combination, composition, or medical device of any of the preceding items, wherein the immune checkpoint inhibitor comprises an inhibitor against at least one factor selected from the group consisting of PD-1, PD-L1, and CTLA-4.

### [Item X7]

The combination, composition, or medical device of any of the preceding items, wherein the immune checkpoint inhibitor comprises at least one agent selected from the group consisting of a PD-1 inhibitor, a PD-L1 inhibitor, and a CTLA-4 inhibitor.

### [Item X8]

The combination, composition, or medical device of any of the preceding items, wherein the cancer or tumor expresses an immune checkpoint factor.

### [Item X9]

The combination, composition, or medical device of any of the preceding items, wherein the cancer expresses PD-L1.

### [Item X10]

The combination, composition, or medical device of any of the preceding items, wherein the immune checkpoint inhibitor comprises at least one selected from the group consisting of nivolumab, pembrolizumab, and atezolizumab.

### [Item X11]

The combination, composition, or medical device of any of the preceding items, wherein the cancer is lung cancer.

### [Item X12]

The combination, composition, or medical device of any of the preceding items, wherein the cancer or tumor has an EGFR gene mutation.

### [Item X13]

The combination, composition, or medical device of any of the preceding items, wherein the treatment, prevention, or prevention of recurrence is performed on a subject who has received therapy with a tyrosine kinase inhibitor.

### [Item X14]

The combination, composition, or medical device of any of the preceding items, wherein the treatment, prevention, or prevention of recurrence is performed on a subject who has not received therapy with an immune checkpoint inhibitor.

### [Item X15]

The combination, composition, or medical device of any of the preceding items, wherein the treatment, prevention, or prevention of recurrence is performed on a subject for whom therapy with an immune checkpoint inhibitor was determined to be ineffective.

### [Item X16]

The combination, composition, or medical device of any of the preceding items, wherein the treatment, prevention, or prevention of recurrence is performed on a subject who received therapy with an immune checkpoint inhibitor which was effective, but subsequently experienced progressive disease (PD).

### [Item X17]

The combination, composition, or medical device of any of the preceding items, wherein the treatment, prevention, or prevention of recurrence is performed on a subject who received therapy with a tyrosine kinase inhibitor and therapy with an immune checkpoint inhibitor which were effective, but subsequently experienced progressive disease (PD) .

### [Item X18]

The combination, composition, or medical device of any of the preceding items, wherein the subject is CXCL10 positive in the subject's tumor.

### [Item X19]

The combination, composition, or medical device of any of the preceding items, wherein the subject is a CD8⁺ T cell^{low} subject.

### [Item X20]

A composition for use in directly activating a dendritic cell, comprising an immunoadjuvant.

### [Item X21]

The composition of any of the preceding items, wherein the immunoadjuvant comprises a human *Mycobacterium tuberculosis* hot water extract or a portion thereof.

### [Item X22]

The composition of any of the preceding items, wherein the activation is determined by measuring an increase in an amount of expression of CD80/86 on a surface of a dendritic cell in the presence of the immunoadjuvant compared to in the absence of the immunoadjuvant.

### [Item X23]

A combination for use in treatment, prevention, or prevention of recurrence of cancer or tumor, comprising:
a) an immune checkpoint inhibitor; and
b) an immunoadjuvant,
characterized in that a) and b) are administered at different times or at the same time.

### [Item X24]

A composition for use in treatment, prevention, or prevention of recurrence of cancer or tumor of a subject, comprising an immunoadjuvant, characterized in that the composition is administered in combination with an immune checkpoint inhibitor.

### [Item X25]

A composition for use in treatment, prevention, or prevention of recurrence of cancer or tumor of a subject, comprising an immune checkpoint inhibitor, characterized in that the composition is administered in combination with an immunoadjuvant.

### [Item X26]

The combination or composition of any of the preceding items, wherein the immunoadjuvant comprises a human *Mycobacterium tuberculosis* hot water extract or a portion thereof.

### [Item X27]

The composition of any of the preceding items, wherein the immune checkpoint inhibitor comprises an inhibitor against at least one factor selected from the group consisting of PD-1, PD-L1, and CTLA-4.

### [Item X28]

The combination or composition of any of the preceding items, wherein the immune checkpoint inhibitor comprises at least one agent selected from the group consisting of a PD-1 inhibitor, a PD-L1 inhibitor, and a CTLA-4 inhibitor.

### [Item X29]

A combination, or a composition or medical device for use in activating a dendritic cell, comprising the composition of any of the preceding items and a radiation therapy providing means.

### [Item X30]

The combination, composition, or medical device of any of the preceding items, wherein the radiation therapy providing means comprises at least one selected from the group consisting of a radiosensitizer, a radiation irradiating device, and a radioactive substance.

### [Item X31]

The combination, composition, or medical device of any of the preceding items, characterized in that the radiation therapy providing means is configured to provide palliative irradiation.

### [Item X32]

The combination, composition, or medical device of any of the preceding items, characterized in that the radiation therapy providing means is configured to irradiate to have an abscopal effect.

### [Item X33]

The combination, composition, or medical device of any of the preceding items, characterized in that the radiation therapy providing means is configured to irradiate a measurable lesion that is present other than a lesion that is a target.

### [Item X34]

The combination, composition, or medical device of any of the preceding items, further comprising a regulatory T cell inhibitor.

### [Item X35]

The combination, composition, or medical device of any of the preceding items, wherein the regulatory T cell inhibitor comprises a COX-2 inhibitor.

### [Item X36]

The combination, composition, or medical device of any of the preceding items, wherein the COX-2 inhibitor is selected from the group consisting of celecoxib, etodolac, meloxicam, nabumetone, zaltoprofen, and lornoxicam.

### [Item X37]

A composition or medical device for use in activating a dendritic cell, comprising a radiation therapy providing means.

### [Item X38]

The composition or medical device of any of the preceding items, wherein the radiation therapy providing means comprises at least one selected from the group consisting of a radiosensitizer, a radiation irradiating device, and a radioactive substance.

### [Item X39]

The composition or medical device of any of the preceding items, wherein the activation is determined by measuring an increase in an amount of expression of CD80/86 on a surface of a dendritic cell in the presence of the immunoadjuvant compared to in the absence of the immunoadjuvant.

### [Item X40]

The composition or medical device of any of the preceding items, characterized in that the radiation therapy providing means is configured to provide palliative irradiation.

### [Item X41]

The composition or medical device of any of the preceding items, characterized in that the radiation therapy providing means is configured to irradiate to have an abscopal effect.

### [Item X42]

The composition or medical device of any of the preceding items, characterized in that the radiation therapy providing means is configured to irradiate a measurable lesion that is present other than a lesion that is a target.

### [Item X43]

A program utilized for cancer treatment or prevention of a patient, characterized by causing a computer to execute:
a step of performing either one or both of first therapy with an immune checkpoint inhibitor and/or a dendritic cell activating agent or means on the patient; and
a step of identifying an optimal therapy program based on a reaction of the patient.

### [Item X44]

The program of any of the preceding items, characterized in that the reaction of the patient is a predetermined change in a medical image of the patient and a measurement value of a tumor marker in the patient.

### [Item X45]

A medicament for use in prevention or treatment of cancer in a patient,
the medicament comprising a combination of an immune checkpoint and a dendritic cell activating agent or means, characterized in that dosage and administration are identified based on information on an optimization identifying marker of the combination obtained from the patient, and a therapeutically or prophylactically effective combination of the immune checkpoint and the dendritic cell activating agent or means is administered based on the identified dosage and administration.

### [Item X46]

A medicament or device for use in treatment, prevention, or prevention of recurrence of cancer, cancer, or tumor of a subject with a specific regimen,
the medicament comprising a combination of an immune checkpoint and a dendritic cell activating agent or means, wherein the regimen comprises:
a) a step of administering an immune checkpoint inhibitor; and
b) a step of administering a dendritic cell activating agent.

### [Item X47]

The medicament or device of any of the preceding items, wherein the subject is a subject who has not received therapy with an immune checkpoint inhibitor or a subject for whom therapy with an immune checkpoint inhibitor alone is ineffective.

### [Item X48]

The medicament or device of any of the preceding items, wherein, in the specific regimen, the immune checkpoint inhibitor:
i) is nivolumab and administered at 240 mg every 2 weeks;
ii) is pembrolizumab and administered at 200 mg at an interval of 3 weeks or at 400 mg at an interval of 6 weeks; or
iii) is atezolizumab and administered at 1200 mg at an interval of 3 weeks.

### [Item X49]

The medicament or device of any of the preceding items, wherein the specific regimen further comprises:
c) a step of performing radiation therapy.

### [Item X50]

The medicament or device of any of the preceding items, characterized in that the radiation therapy is palliative irradiation other than brain metastasis, and irradiation is performed 10 fractions at 3 Gy or 20 to 25 fractions at 2 Gy.

### [Item X51]

The medicament or device of any of the preceding items, wherein the dendritic cell activating agent or means comprises Extract Z that is administered by aspirating a liquid medicine into a 1 mL syringe and subcutaneously injecting 0.05 mL thereof twice a week.

### [Item XA1]

A method for treatment, prevention, or prevention of recurrence of cancer or tumor in a subject, comprising administering:
a) an effective amount of an immune checkpoint inhibitor; and
b) an effective amount of a direct dendritic cell activating agent or means
to the subject at different times or at the same time.

### [Item XA2]

A method for treatment, prevention, or prevention of recurrence of cancer or tumor in a subject, comprising administering or using an effective amount of a direct dendritic cell activating agent or means to the subject, wherein administration of the direct dendritic cell activating agent or means comprises administration or use in combination with an effective amount of an immune checkpoint inhibitor.

### [Item XA3]

A method for treatment, prevention, or prevention of recurrence of cancer or tumor of a subject, comprising administering an effective amount of an immune checkpoint inhibitor to the subject, wherein administration of the immune checkpoint inhibitor comprises administration or use in combination with an effective amount of a direct dendritic cell activating agent or means.

### [Item XA4]

The method of any of the preceding items, wherein the direct dendritic cell activating agent or means comprises at least one selected from the group consisting of a radiation therapy providing means and an immunoadjuvant.

### [Item XA5]

The method of any of the preceding items, wherein the direct dendritic cell activating agent or means comprises a radiation therapy providing means and an immunoadjuvant.

### [Item XA6]

The method of any of the preceding items, wherein the immune checkpoint inhibitor comprises an inhibitor against at least one factor selected from the group consisting of PD-1, PD-L1, and CTLA-4.

### [Item XA7]

The method of any of the preceding items, wherein the immune checkpoint inhibitor comprises at least one agent selected from the group consisting of a PD-1 inhibitor, a PD-L1 inhibitor, and a CTLA-4 inhibitor.

### [Item XA8]

The method of any of the preceding items, wherein the cancer or tumor expresses an immune checkpoint factor.

### [Item XA9]

The method of any of the preceding items, wherein the cancer expresses PD-L1.

### [Item XA10]

The method of any of the preceding items, wherein the immune checkpoint inhibitor comprises at least one selected from the group consisting of nivolumab, pembrolizumab, and atezolizumab.

### [Item XA11]

The method of any of the preceding items, wherein the cancer is lung cancer.

### [Item XA12]

The method of any of the preceding items, wherein the cancer or tumor has an EGFR gene mutation.

### [Item XA13]

The method of any of the preceding items, wherein the method is performed on a subject who has received therapy with a tyrosine kinase inhibitor.

### [Item XA14]

The method of any of the preceding items, wherein the method is performed on a subject who has not received therapy with an immune checkpoint inhibitor.

### [Item XA15]

The method of any of the preceding items, wherein the method is performed on a subject for whom therapy with an immune checkpoint inhibitor was determined to be ineffective.

### [Item XA16]

The method of any of the preceding items, wherein the method is performed on a subject who received therapy with an immune checkpoint inhibitor which was effective, but subsequently experienced progressive disease (PD).

### [Item XA17]

The method of any of the preceding items, wherein the method is performed on a subject who received therapy with a tyrosine kinase inhibitor and therapy with an immune checkpoint inhibitor which were effective, but subsequently experienced progressive disease (PD).

### [Item XA18]

The method of any of the preceding items, wherein the subject is CXCL10 positive in the subject's tumor.

### [Item XA19]

The method of any of the preceding items, wherein the subject is a CD8⁺ T cell^{low} subject.

### [Item XA20]

A method for directly activating a dendritic cell in a subject, comprising administering an effective amount of an immunoadjuvant to the subject.

### [Item XA21]

The method of any of the preceding items, wherein the immunoadjuvant comprises a human *Mycobacterium tuberculosis* hot water extract or a portion thereof.

### [Item XA22]

The method of any of the preceding items, wherein the activation is determined by measuring an increase in an amount of expression of CD80/86 on a surface of a dendritic cell in the presence of the immunoadjuvant compared to in the absence of the immunoadjuvant.

### [Item XA23]

A method for treatment, prevention, or prevention of recurrence of cancer or tumor in a subject, comprising administering:
a) an effective amount of an immune checkpoint inhibitor; and
b) an effective amount of an immunoadjuvant
to the subject at different times or at the same time.

### [Item XA24]

A method for treatment, prevention, or prevention of recurrence of cancer or tumor in a subject, comprising administering an effective amount of an immunoadjuvant to the subject, wherein administration of the immunoadjuvant comprises administration in combination with an effective amount of an immune checkpoint inhibitor.

### [Item XA25]

A method for treatment, prevention, or prevention of recurrence of cancer or tumor of a subject, comprising administering an immune checkpoint inhibitor to the subject, wherein the immune checkpoint inhibitor is administered in combination with an immunoadjuvant.

### [Item XA26]

The method of any of the preceding items, wherein the immunoadjuvant comprises a human *Mycobacterium tuberculosis* hot water extract or a portion thereof.

### [Item XA27]

The method of any of the preceding items, wherein the immune checkpoint inhibitor comprises an inhibitor against at least one factor selected from the group consisting of PD-1, PD-L1, and CTLA-4.

### [Item XA28]

The method of any of the preceding items, wherein the immune checkpoint inhibitor comprises at least one agent selected from the group consisting of a PD-1 inhibitor, a PD-L1 inhibitor, and a CTLA-4 inhibitor.

### [Item XA29]

A method for activating a dendritic cell, wherein a radiation therapy providing means is further used.

### [Item XA30]

The method of any of the preceding items, wherein the radiation therapy providing means comprises at least one selected from the group consisting of a radiosensitizer, a radiation irradiating device, and a radioactive substance.

### [Item XA31]

The method of any of the preceding items, wherein the radiation therapy providing means is configured to provide palliative irradiation.

### [Item XA32]

The method of any of the preceding items, wherein the radiation therapy providing means is configured to irradiate to have an abscopal effect.

### [Item XA33]

The method of any of the preceding items, wherein the radiation therapy providing means is configured to irradiate a measurable lesion that is present other than a lesion that is a target.

### [Item XA34]

The method of any of the preceding items, wherein a regulatory T cell inhibitor is further administered.

### [Item XA35]

The method of any of the preceding items, wherein the regulatory T cell inhibitor comprises a COX-2 inhibitor.

### [Item XA36]

The method of any of the preceding items, wherein the COX-2 inhibitor is selected from the group consisting of celecoxib, etodolac, meloxicam, nabumetone, zaltoprofen, and lornoxicam.

### [Item XA37]

A method for activating a dendritic cell, comprising using a radiation therapy providing means for a subject.

### [Item XA38]

The method of any of the preceding items, wherein the radiation therapy providing means comprises at least one selected from the group consisting of a radiosensitizer, a radiation irradiating device, and a radioactive substance.

### [Item XA39]

The method of any of the preceding items, wherein the activation is determined by measuring an increase in an amount of expression of CD80/86 on a surface of a dendritic cell in the presence of the immunoadjuvant compared to in the absence of the immunoadjuvant.

### [Item XA40]

The method of any of the preceding items, wherein the radiation therapy providing means is configured to provide palliative irradiation.

### [Item XA41]

The method of any of the preceding items, wherein the radiation therapy providing means is configured to irradiate to have an abscopal effect.

### [Item XA42]

The method of any of the preceding items, wherein the radiation therapy providing means is configured to irradiate a measurable lesion that is present other than a lesion that is a target.

### [Item XA43]

A method for treatment or prevention of cancer of a patient, comprising causing a computer to execute:
a step of performing either one or both of first therapy with an effective amount of an immune checkpoint inhibitor and/or an effective amount of a dendritic cell activating agent or means on the patient; and
a step of identifying an optimal therapy program based on a reaction of the patient.

### [Item XA44]

The method of any of the preceding items, characterized in that the reaction of the patient is a predetermined change in a medical image of the patient and a measurement value of a tumor marker in the patient.

### [Item XA45]

A method for prevention or treatment of cancer in a patient, comprising administering a combination of an effective amount of an immune checkpoint and an effective amount of a dendritic cell activating agent or means,
wherein, based on information on an optimization identifying marker of the combination obtained from the patient, dosage and administration of a therapeutically or prophylactically effective combination of the immune checkpoint and the dendritic cell activating agent or means are identified, and the combination is administered based on the identified dosage and administration.

### [Item XA46]

A method for treatment, prevention, or prevention of recurrence of cancer or tumor of a subject with a specific regimen,
the method comprising administering a combination of an effective amount of an immune checkpoint and an effective amount of a dendritic cell activating agent or means, wherein the regimen comprises:
a) a step of administering an effective amount of an immune checkpoint inhibitor; and
b) a step of administering an effective amount of a dendritic cell activating agent.

### [Item XA47]

The method of any of the preceding items, wherein the subject is a subject who has not received therapy with an immune checkpoint inhibitor or a subject for whom therapy with an immune checkpoint inhibitor alone is ineffective.

### [Item XA48]

The method of any of the preceding items, wherein, in the specific regimen, the immune checkpoint inhibitor:
i) is nivolumab and administered at 240 mg every 2 weeks;
ii) is pembrolizumab and administered at 200 mg at an interval of 3 weeks or at 400 mg at an interval of 6 weeks; or
iii) is atezolizumab and administered at 1200 mg at an interval of 3 weeks.

### [Item XA49]

The method of any of the preceding items, wherein the specific regimen further comprises:
c) a step of performing radiation therapy.

### [Item XA50]

The method of any of the preceding items, wherein the radiation therapy is palliative irradiation other than brain metastasis, and irradiation is performed 10 fractions at 3 Gy or 20 to 25 fractions at 2 Gy.

### [Item XA51]

The method of any of the preceding items, wherein the dendritic cell activating agent or means comprises Extract Z that is administered by aspirating a liquid medicine into a 1 mL syringe and subcutaneously injecting 0.05 mL thereof twice a week.

### [Item XB1]

Use of a) an immune checkpoint inhibitor and b) a direct dendritic cell activating agent or means in the manufacture of a medicament for treatment, prevention, or prevention of recurrence of cancer or tumor.

### [Item XB2]

Use of a direct dendritic cell activating agent or means in the manufacture of a medicament that is used in combination with an immune checkpoint inhibitor for therapy, prevention, or prevention of recurrence of cancer or tumor of a subject.

### [Item XB3]

Use of an immune checkpoint inhibitor in the manufacture of a medicament that is used in combination with a direct dendritic cell activating agent or means for treatment, prevention, or prevention of recurrence of cancer or tumor of a subject.

### [Item XB4]

The use of any of the preceding items, wherein the direct dendritic cell activating agent or means comprises at least one selected from the group consisting of a radiation therapy providing means and an immunoadjuvant.

### [Item XB5]

The use of any of the preceding items, wherein the direct dendritic cell activating agent or means comprises a radiation therapy providing means and an immunoadjuvant.

### [Item XB6]

The use of any of the preceding items, wherein the immune checkpoint inhibitor comprises an inhibitor against at least one factor selected from the group consisting of PD-1, PD-L1, and CTLA-4.

### [Item XB7]

The use of any of the preceding items, wherein the immune checkpoint inhibitor comprises at least one agent selected from the group consisting of a PD-1 inhibitor, a PD-L1 inhibitor, and a CTLA-4 inhibitor.

### [Item XB8]

The use of any of the preceding items, wherein the cancer or tumor expresses an immune checkpoint factor.

### [Item XB9]

The use of any of the preceding items, wherein the cancer expresses PD-L1.

### [Item XB10]

The use of any of the preceding items, wherein the immune checkpoint inhibitor comprises at least one selected from the group consisting of nivolumab, pembrolizumab, and atezolizumab.

### [Item XB11]

The use of any of the preceding items, wherein the cancer is lung cancer.

### [Item XB12]

The use of any of the preceding items, wherein the cancer or tumor has an EGFR gene mutation.

### [Item XB13]

The use of any of the preceding items, wherein the treatment, prevention, or prevention of recurrence is performed on a subject who has received therapy with a tyrosine kinase inhibitor.

### [Item XB14]

The use of any of the preceding items, wherein the treatment, prevention, or prevention of recurrence is performed on a subject who has not received therapy with an immune checkpoint inhibitor.

### [Item XB15]

The use of any of the preceding items, wherein the treatment, prevention, or prevention of recurrence is performed on a subject for whom therapy with an immune checkpoint inhibitor was determined to be ineffective.

### [Item XB16]

The use of any of the preceding items, wherein the treatment, prevention, or prevention of recurrence is performed on a subject who received therapy with an immune checkpoint inhibitor which was effective, but subsequently experienced progressive disease (PD).

### [Item XB17]

The use of any of the preceding items, wherein the treatment, prevention, or prevention of recurrence is performed on a subject who received therapy with a tyrosine kinase inhibitor and therapy with an immune checkpoint inhibitor which were effective, but subsequently experienced progressive disease (PD).

### [Item XB18]

The use of any of the preceding items, wherein the subject is CXCL10 positive in the subject's tumor.

### [Item XB19]

The use of any of the preceding items, wherein the subject is a CD8⁺ T cell^{low} subject.

### [Item XB20]

Use of an immunoadjuvant in the manufacture of a medicament for directly activating a dendritic cell.

### [Item XB21]

The use of any of the preceding items, wherein the immunoadjuvant comprises a human *Mycobacterium tuberculosis* hot water extract or a portion thereof.

### [Item XB22]

The use of any of the preceding items, wherein the activation is determined by measuring an increase in an amount of expression of CD80/86 on a surface of a dendritic cell in the presence of the immunoadjuvant compared to in the absence of the immunoadjuvant.

### [Item XB23]

Use of a) an immune checkpoint inhibitor and b) an immunoadjuvant in the manufacture of a medicament for treatment, prevention, or prevention of recurrence of cancer or tumor.

### [Item XB24]

Use of an immunoadjuvant in the manufacture of a medicament that is used in combination with an immune checkpoint inhibitor for treatment, prevention, or prevention of recurrence of cancer or tumor.

### [Item XB25]

Use of an immune checkpoint inhibitor in the manufacture of a medicament that is used in combination with an immunoadjuvant for treatment, prevention, or prevention of recurrence of cancer or tumor.

### [Item XB26]

The use of any of the preceding items, wherein the immunoadjuvant comprises a human *Mycobacterium tuberculosis* hot water extract or a portion thereof.

### [Item XB27]

The use of any of the preceding items, wherein the immune checkpoint inhibitor comprises an inhibitor against at least one factor selected from the group consisting of PD-1, PD-L1, and CTLA-4.

### [Item XB28]

The use of any of the preceding items, wherein the immune checkpoint inhibitor comprises at least one agent selected from the group consisting of a PD-1 inhibitor, a PD-L1 inhibitor, and a CTLA-4 inhibitor.

### [Item XB29]

The use of any of the preceding items, characterized in that a radiation therapy providing means is further used for the subject.

### [Item XB30]

The use of any of the preceding items, wherein the radiation therapy providing means comprises at least one selected from the group consisting of a radiosensitizer, a radiation irradiating device, and a radioactive substance.

### [Item XB31]

The use of any of the preceding items, characterized in that the radiation therapy providing means is configured to provide palliative irradiation.

### [Item XB32]

The use of any of the preceding items, characterized in that the radiation therapy providing means is configured to irradiate to have an abscopal effect.

### [Item XB33]

The use of any of the preceding items, characterized in that the radiation therapy providing means is configured to irradiate a measurable lesion that is present other than a lesion that is a target.

### [Item XB34]

The use of any of the preceding items, characterized in that a regulatory T cell inhibitor is further administered to the subject.

### [Item XB35]

The use of any of the preceding items, wherein the regulatory T cell inhibitor comprises a COX-2 inhibitor.

### [Item XB36]

The use of any of the preceding items, wherein the COX-2 inhibitor is selected from the group consisting of celecoxib, etodolac, meloxicam, nabumetone, zaltoprofen, and lornoxicam.

### [Item XB37]

Use of a radiation therapy providing means in the manufacture of a medicament for activating a dendritic cell.

### [Item XB38]

The use of any of the preceding items, wherein the radiation therapy providing means comprises at least one selected from the group consisting of a radiosensitizer, a radiation irradiating device, and a radioactive substance.

### [Item XB39]

The use of any of the preceding items, wherein the activation is determined by measuring an increase in an amount of expression of CD80/86 on a surface of a dendritic cell in the presence of the immunoadjuvant compared to in the absence of the immunoadjuvant.

### [Item XB40]

The use of any of the preceding items, characterized in that the radiation therapy providing means is configured to provide palliative irradiation.

### [Item XB41]

The use of any of the preceding items, characterized in that the radiation therapy providing means is configured to irradiate to have an abscopal effect.

### [Item XB42]

The use of any of the preceding items, characterized in that the radiation therapy providing means is configured to irradiate a measurable lesion that is present other than a lesion that is a target.

### [Item XB43]

A program utilized for cancer treatment or prevention of a patient, characterized by causing a computer to execute:
a step of performing either one or both of first therapy with an immune checkpoint inhibitor and/or a dendritic cell activating agent or means on the patient; and
a step of identifying an optimal therapy program based on a reaction of the patient.

### [Item XB44]

The program of any of the preceding items, characterized in that the reaction of the patient is a predetermined change in a medical image of the patient and a measurement value of a tumor marker in the patient.

### [Item XB45]

Use of a combination of an immune checkpoint and a dendritic cell activating agent or means in the manufacture of a medicament for prevention or treatment of cancer in a patient,
wherein the medicament is characterized in that dosage and administration are identified based on information on an optimization identifying marker of the combination obtained from the patient, and a therapeutically or prophylactically effective combination of the immune checkpoint and the dendritic cell activating agent or means is administered based on the identified dosage and administration.

### [Item XB46]

Use of an immune checkpoint inhibitor and a dendritic cell activating agent in the manufacture of a medicament for treatment, prevention, or prevention of recurrence of cancer or tumor of a subject with a specific regimen.

### [Item XB47]

The use of any of the preceding items, wherein the subject is a subject who has not received therapy with an immune checkpoint inhibitor or a subject for whom therapy with an immune checkpoint inhibitor alone is ineffective.

### [Item XB48]

The use of any of the preceding items, wherein, in the specific regimen, the immune checkpoint inhibitor:
i) is nivolumab and administered at 240 mg every 2 weeks;
ii) is pembrolizumab and administered at 200 mg at an interval of 3 weeks or at 400 mg at an interval of 6 weeks; or
iii) is atezolizumab and administered at 1200 mg at an interval of 3 weeks.

### [Item XB49]

The use of any of the preceding items, wherein the specific regimen further comprises:
c) a step of performing radiation therapy.

### [Item XB50]

The use of any of the preceding items, characterized in that the radiation therapy is palliative irradiation other than brain metastasis, and irradiation is performed 10 fractions at 3 Gy or 20 to 25 fractions at 2 Gy.

### [Item XB51]

The use of any of the preceding items, wherein the dendritic cell activating agent or means comprises Extract Z that is administered by aspirating a liquid medicine into a 1 mL syringe and subcutaneously injecting 0.05 mL thereof twice a week.

### [Item XC1]

An immune checkpoint inhibitor for use in treatment, prevention, or prevention of recurrence of cancer or tumor, characterized by being used in combination with a direct dendritic cell activating agent or means.

### [Item XC2]

A direct dendritic cell activating agent or means for use in treatment, prevention, or prevention of recurrence of cancer or tumor of a subject, characterized by being administered in combination with an immune checkpoint inhibitor.

### [Item XC3]

An immune checkpoint inhibitor for use in treatment, prevention, or prevention of recurrence of cancer or tumor of a subject, characterized by being administered in combination with a direct dendritic cell activating agent or means.

### [Item XC4]

The immune checkpoint inhibitor or direct dendritic cell activating agent or means of any of the preceding items, wherein the direct dendritic cell activating agent or means comprises at least one selected from the group consisting of a radiation therapy providing means and an immunoadjuvant.

### [Item XC5]

The immune checkpoint inhibitor or direct dendritic cell activating agent or means of any of the preceding items, wherein the direct dendritic cell activating agent or means comprises a radiation therapy providing means and an immunoadjuvant.

### [Item XC6]

The immune checkpoint inhibitor or direct dendritic cell activating agent or means of any of the preceding items, wherein the immune checkpoint inhibitor comprises an inhibitor against at least one factor selected from the group consisting of PD-1, PD-L1, and CTLA-4.

### [Item XC7]

The immune checkpoint inhibitor or direct dendritic cell activating agent or means of any of the preceding items, wherein the immune checkpoint inhibitor comprises at least one agent selected from the group consisting of a PD-1 inhibitor, a PD-L1 inhibitor, and a CTLA-4 inhibitor.

### [Item XC8]

The immune checkpoint inhibitor or direct dendritic cell activating agent or means of any of the preceding items, wherein the cancer or tumor expresses an immune checkpoint factor.

### [Item XC9]

The immune checkpoint inhibitor or direct dendritic cell activating agent or means of any of the preceding items, wherein the cancer expresses PD-L1.

### [Item XC10]

The immune checkpoint inhibitor or direct dendritic cell activating agent or means of any of the preceding items, wherein the immune checkpoint inhibitor comprises at least one selected from the group consisting of nivolumab, pembrolizumab, and atezolizumab.

### [Item XC11]

The immune checkpoint inhibitor or direct dendritic cell activating agent or means of any of the preceding items, wherein the cancer is lung cancer.

### [Item XC12]

The immune checkpoint inhibitor or direct dendritic cell activating agent or means of any of the preceding items, wherein the cancer or tumor has an EGFR gene mutation.

### [Item XC13]

The immune checkpoint inhibitor or direct dendritic cell activating agent or means of any of the preceding items, wherein the treatment, prevention, or prevention of recurrence is performed on a subject who has received therapy with a tyrosine kinase inhibitor.

### [Item XC14]

The immune checkpoint inhibitor or direct dendritic cell activating agent or means of any of the preceding items, wherein the treatment, prevention, or prevention of recurrence is performed on a subject who has not received therapy with an immune checkpoint inhibitor.

### [Item XC15]

The immune checkpoint inhibitor or direct dendritic cell activating agent or means of any of the preceding items, wherein the treatment, prevention, or prevention of recurrence is performed on a subject for whom therapy with an immune checkpoint inhibitor was determined to be ineffective.

### [Item XC16]

The immune checkpoint inhibitor or direct dendritic cell activating agent or means of any of the preceding items, wherein the treatment, prevention, or prevention of recurrence is performed on a subject who received therapy with an immune checkpoint inhibitor which was effective, but subsequently experienced progressive disease (PD).

### [Item XC17]

The immune checkpoint inhibitor or direct dendritic cell activating agent or means of any of the preceding items, wherein the treatment, prevention, or prevention of recurrence is performed on a subject who received therapy with a tyrosine kinase inhibitor and treatment with an immune checkpoint inhibitor which were effective, but subsequently experienced progressive disease (PD).

### [Item XC18]

The immune checkpoint inhibitor or direct dendritic cell activating agent or means of any of the preceding items, wherein the subject is CXCL10 positive in the subject's tumor.

### [Item XC19]

The immune checkpoint inhibitor or direct dendritic cell activating agent or means of any of the preceding items, wherein the subject is a CD8⁺ T cell^{low} subject.

### [Item XC20]

An immunoadjuvant for use in directly activating a dendritic cell.

### [Item XC21]

The immunoadjuvant of any of the preceding items, comprising a human *Mycobacterium tuberculosis* hot water extract or a portion thereof.

### [Item XC22]

The immunoadjuvant of any of the preceding items, wherein the activation is determined by measuring an increase in an amount of expression of CD80/86 on a surface of a dendritic cell in the presence of the immunoadjuvant compared to in the absence of the immunoadjuvant.

### [Item XC23]

An immune checkpoint inhibitor for use in treatment, prevention, or prevention of recurrence of cancer or tumor, characterized by being administered in combination with an immunoadjuvant.

### [Item XC24]

An immunoadjuvant for use in treatment, prevention, or prevention of recurrence of cancer or tumor of a subject, characterized by being administered in combination with an immune checkpoint inhibitor.

### [Item XC25]

An immune checkpoint inhibitor for use in treatment, prevention, or prevention of recurrence of cancer or tumor of a subject, characterized by being administered in combination with an immunoadjuvant.

### [Item XC26]

The immunoadjuvant or immune checkpoint inhibitor of any of the preceding items, wherein the immunoadjuvant comprises a human *Mycobacterium tuberculosis* hot water extract or a portion thereof.

### [Item XC27]

The immunoadjuvant or immune checkpoint inhibitor of any of the preceding items, wherein the immune checkpoint inhibitor comprises an inhibitor against at least one factor selected from the group consisting of PD-1, PD-L1, and CTLA-4.

### [Item XC28]

The immunoadjuvant or immune checkpoint inhibitor of any of the preceding items, wherein the immune checkpoint inhibitor comprises at least one agent selected from the group consisting of a PD-1 inhibitor, a PD-L1 inhibitor, and a CTLA-4 inhibitor.

### [Item XC29]

The immunoadjuvant or immune checkpoint inhibitor of any of the preceding items, characterized in that a radiation therapy providing means is further administered.

### [Item XC30]

The immunoadjuvant or immune checkpoint inhibitor of any of the preceding items, wherein the radiation therapy providing means comprises at least one selected from the group consisting of a radiosensitizer, a radiation irradiating device, and a radioactive substance.

### [Item XC31]

The immunoadjuvant or immune checkpoint inhibitor of any of the preceding items, characterized in that the radiation therapy providing means is configured to provide palliative irradiation.

### [Item XC32]

The immunoadjuvant or immune checkpoint inhibitor of any of the preceding items, characterized in that the radiation therapy providing means is configured to irradiate to have an abscopal effect.

### [Item XC33]

The immunoadjuvant or immune checkpoint inhibitor of any of the preceding items, characterized in that the radiation therapy providing means is configured to irradiate a measurable lesion that is present other than a lesion that is a target.

### [Item XC34]

The immunoadjuvant or immune checkpoint inhibitor of any of the preceding items, further comprising a regulatory T cell inhibitor.

### [Item XC35]

The immunoadjuvant or immune checkpoint inhibitor of any of the preceding items, wherein the regulatory T cell inhibitor comprises a COX-2 inhibitor.

### [Item XC36]

The immunoadjuvant or immune checkpoint inhibitor of any of the preceding items, wherein the COX-2 inhibitor is selected from the group consisting of celecoxib, etodolac, meloxicam, nabumetone, zaltoprofen, and lornoxicam.

### [Item XC37]

A radiation therapy providing means for use in activating a dendritic cell.

### [Item XC38]

The radiation therapy providing means of any of the preceding items, comprising at least one selected from the group consisting of a radiosensitizer, a radiation irradiating device, and a radioactive substance.

### [Item XC39]

The radiation therapy providing means of any of the preceding items, wherein the activation is determined by measuring an increase in an amount of expression of CD80/86 on a surface of a dendritic cell in the presence of the immunoadjuvant compared to in the absence of the immunoadjuvant.

### [Item XC40]

The radiation therapy providing means of any of the preceding items, characterized by being configured to provide palliative irradiation.

### [Item XC41]

The radiation therapy providing means of any of the preceding items, characterized by being configured to irradiate to have an abscopal effect.

### [Item XC42]

The radiation therapy providing means of any of the preceding items, characterized by being configured to irradiate a measurable lesion that is present other than a lesion that is a target.

### [Item XC43]

A program utilized for cancer treatment or prevention of a patient, characterized by causing a computer to execute:
a step of performing either one or both of first therapy with an immune checkpoint inhibitor and/or a dendritic cell activating agent or means on the patient; and
a step of identifying an optimal therapy program based on a reaction of the patient.

### [Item XC44]

The program of any of the preceding items, characterized in that the reaction of the patient is a predetermined change in a medical image of the patient and a measurement value of a tumor marker in the patient.

### [Item XC45]

An immune checkpoint inhibitor for use in prevention or treatment of cancer in a patient, the immune checkpoint inhibitor characterized by being administered in combination with a dendritic cell activating agent or means, characterized in that dosage and administration are identified based on information on an optimization identifying marker of the combination obtained from the patient, and a therapeutically or prophylactically effective combination of the immune checkpoint and the dendritic cell activating agent or means is administered based on the identified dosage and administration.

### [Item XC45A]

A dendritic cell activating agent or means for use in prevention or treatment of cancer in a patient, the dendritic cell activating agent or means characterized by being administered in combination with an immune checkpoint inhibitor, characterized in that dosage and administration are identified based on information on an optimization identifying marker of the combination obtained from the patient, and a therapeutically or prophylactically effective combination of the immune checkpoint and the dendritic cell activating agent or means is administered based on the identified dosage and administration.

### [Item XC46]

An immune checkpoint inhibitor for use in treatment, prevention, or prevention of recurrence of cancer, cancer, or tumor of a subject with a specific regimen, wherein the regimen comprises a step of administering the immune checkpoint inhibitor in combination with a dendritic cell activating agent.

### [Item XC46A]

A dendritic cell activating agent for use in treatment, prevention, or prevention of recurrence of cancer, cancer, or tumor of a subject with a specific regimen, wherein the regimen comprises a step of administering the dendritic cell activating agent in combination with an immune checkpoint inhibitor.

### [Item XC47]

The immune checkpoint inhibitor or dendritic cell activating agent of any of the preceding items, wherein the subject is a subject who has not received therapy with an immune checkpoint inhibitor or a subject for whom therapy with an immune checkpoint inhibitor alone is ineffective.

### [Item XC48]

The immune checkpoint inhibitor or dendritic cell activating agent of any of the preceding items, wherein, in the specific regimen, the immune checkpoint inhibitor:
i) is nivolumab and administered at 240 mg every 2 weeks;
ii) is pembrolizumab and administered at 200 mg at an interval of 3 weeks or at 400 mg at an interval of 6 weeks; or
iii) is atezolizumab and administered at 1200 mg at an interval of 3 weeks.

### [Item XC49]

The immune checkpoint inhibitor or dendritic cell activating agent of any of the preceding items, wherein the specific regimen further comprises:
c) a step of performing radiation therapy.

### [Item XC50]

The immune checkpoint inhibitor or dendritic cell activating agent of any of the preceding items, characterized in that the radiation therapy is palliative irradiation other than brain metastasis, and irradiation is performed 10 fractions at 3 Gy or 20 to 25 fractions at 2 Gy.

### [Item XC51]

The immune checkpoint inhibitor or dendritic cell activating agent of any of the preceding items, wherein the dendritic cell activating agent or means comprises Extract Z that is administered by aspirating a liquid medicine into a 1 mL syringe and subcutaneously injecting 0.05 mL thereof twice a week.

In one embodiment, the method for cancer treatment of the present disclosure is a method for treating cancer, characterized by combining therapy with an immune checkpoint inhibitor and therapy for increasing sensitivity to the immune checkpoint inhibitor.

In one embodiment, the method for cancer treatment of the present disclosure is a method for treating cancer of a patient, characterized by having a first step of administering an immune checkpoint inhibitor to the patient and a second step of performing treatment for increasing sensitivity to the immune checkpoint inhibitor on the patient.

In one embodiment, the method for cancer treatment of the present disclosure is characterized by performing the second step at the same time as or at a different time from the first step.

In one embodiment, the method for cancer treatment of the present disclosure is characterized in that the immune checkpoint inhibitor is nivolumab, pembrolizumab, or atezolizumab.

In one embodiment, the medicament of the present disclosure is a medicament for cancer treatment which comprises an immune checkpoint inhibitor and is used in a regimen in which the medicament is administered in combination with radiation therapy, characterized in that the regimen increases sensitivity to the immune checkpoint inhibitor by irradiation of the radiation.

In one embodiment, the medicament of the present disclosure is a medicament for cancer treatment which comprises an immune checkpoint inhibitor and Extract Z and is used in a regimen in which the immune checkpoint inhibitor and the Extract Z are administered at different times or at the same time, characterized in that the regimen increases sensitivity to the immune checkpoint inhibitor by administration of the Extract Z.

In one embodiment, the program of the present disclosure is a program utilized for cancer treatment of a patient, characterized by causing a computer to execute: a step of identifying a first period during which either one of first therapy with an immune checkpoint inhibitor and second therapy for increasing sensitivity to the immune checkpoint inhibitor is performed on the patient; and a step of identifying a second period during which the other or both of the first therapy and the second therapy is/are performed on the patient when the test result with respect to the patient shows a predetermined change.

In one embodiment, the program of the present disclosure is characterized in that the predetermined change is a predetermined change in a medical image of the patient and a measurement value of a tumor marker in the patient.

In one embodiment, the program of the present disclosure is characterized in that the second therapy comprises at least one of radiation irradiation and combined use of administration of Extract Z and radiation irradiation.

In one embodiment, the program of the present disclosure is characterized in that the immune checkpoint inhibitor is nivolumab, pembrolizumab, or atezolizumab.

In one embodiment, the present disclosure combines therapy with an immune checkpoint inhibitor (first therapy) and therapy for increasing sensitivity to the immune checkpoint inhibitor (second therapy) to perform cancer therapy, and eliminates or reduces cancer tissue by the synergistic action of these two therapies.

In one embodiment, the present disclosure performs therapy for increasing sensitivity to an immune checkpoint inhibitor (second therapy) before or while performing therapy with the immune checkpoint inhibitor (first therapy). It is intended to efficiently increase the sensitivity to the immune checkpoint inhibitor.

In one embodiment, the present disclosure identifies a first period during which either one of first therapy with an immune checkpoint inhibitor and second therapy for increasing sensitivity to the immune checkpoint inhibitor is performed, and when the test result with respect to the patient shows a predetermined change (e.g., when cancer tissue does not reduce according to image diagnosis, and in addition, a measurement value of a tumor marker increases), identifies a second period during which the other or both of the first therapy and the second therapy is/are performed. Efficient synergistic action of the first therapy and the second therapy is intended.

In one embodiment, the present disclosure performs radiation irradiation or combined use of administration of Extract Z and radiation irradiation alone or performs a combination of radiation irradiation and combined use of administration of Extract Z and radiation irradiation as treatment for increasing sensitivity to an immune checkpoint inhibitor to improve the sensitivity to the immune checkpoint inhibitor.

In one embodiment, the present disclosure uses nivolumab, pembrolizumab, or atezolizumab that is an antibody against PD-1 of a T cell as an immune checkpoint inhibitor to inhibit an immune checkpoint of a T cell and a cancer cell.

The present disclosure is intended so that one or more of the aforementioned features can be provided not only as the explicitly disclosed combinations, but also as other combinations thereof. Additional embodiments and advantages of the present disclosure are recognized by those skilled in the art by reading and understanding the following detailed description as needed.

### [Advantageous Effects]

The present disclosure can unexpectedly activate a dendritic cell. The present disclosure also can provide a method for effectively treating or preventing cancer or tumor by using an immunoadjuvant or the like.

### [Brief Description of Drawings]

[Figure 1] Figure 1 is a block diagram showing the configuration of a system that utilizes a program in the present embodiment.
[Figure 2] Figure 2 is a flowchart showing the treatment procedure in the present embodiment.
[Figure 3] Figure 3 is a diagram describing the change over time in the therapeutic approach in Example 1.
[Figure 4] Figure 4 is a diagram describing the change over time in the therapeutic approach in Example 2.
[Figure 5] Figure 5 is a diagram describing the change over time in the tumor marker measurement value and the therapeutic approach in Example 3.
[Figure 6] Figure 6 is a diagram describing the change over time in the tumor marker measurement value and the therapeutic approach in Example 4.
[Figure 7] Figure 7 is a diagram showing enhancement of CXCL10 production by an immunoadjuvant in Example 13.
[Figure 8] Figure 8 is a diagram showing the effect of promoting infiltration of CD8⁺ T cells into tumor in Example 15.
[Figure 9] Figure 9 is a diagram showing PD-L1 expression in cultured Sq-1979 cells in Example 20.
[Figure 10] Figure 10 is a diagram describing the change over time in the therapeutic approach in Example 23.
[Figure 11] Figure 11 is a diagram describing the change over time in the tumor marker measurement value and the therapeutic approach in Example 23.

### [Description of Embodiments]

The present disclosure is described hereinafter while showing some of the best modes thereof. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

### (Definitions)

The terms used herein are described hereinafter.

As used herein, an "immune checkpoint inhibitor" is a substance capable of disabling suppression of activation of a T cell by an immune checkpoint molecule by binding to an immune checkpoint molecule or a ligand thereof to inhibit immunosuppressive signaling. Specific examples include inhibitors against PD-1, PD-L1, PD-L2, CTLA-4, LAG-3, TIM-1, TIM-3, TIM-4, VISTA, BTLA, TIGIT, A2AR, 4-1BB, 4-1BBL, 2B4 (CD244), KIR family receptors, B7.1, B7.2, B7-H2, B7-H3, B7-H4, B7-H6, BATE, CD39, CD40, CD47, CD48, CD73, CD94/NKG2A, CD96, CD160, CD200, CD200R, CD274, butyrophilins, CEACAM1, CSF-1R, DcR3, EDO, Foxp1, GARP, GITR, gp49B, HHLA2, HVEM, ICOS, IDO, ILT-2, ILT-4, LAIR-1, MAFB, MICA./B, NKG2A/HLA-E, NR4A2, OCT-2, OX-40, PIR-B, Rara (retinoic acid receptor alpha), SIRP, TDO, TLR3, and TNFR. Preferred examples include inhibitors against PD-1, PD-L1, and CTLA-4. An anti-PD-1 antibody, which is one of PD-1 inhibitors, binds to PD-1 on a T cell to inhibit binding between PD-1 and PD-L1, thereby blocking suppressive signaling and maintaining activation of the T cell. An anti-PD-Ll antibody, which is one of PD-L1 inhibitors, binds to PD-L1 expressed on a cancer cell or an antigen-presenting cell to inhibit interaction with PD-1 on a T cell. As a result, suppressive signaling to the T cell is inhibited, and activation of the T cell is maintained. An anti-CTLA-4 antibody, which is one of CTLA-4 inhibitors, competes with a CD28 ligand on a dendritic cell and blocks a suppressive signal of an immune cell via CD28 to maintain activation of a T cell. Examples of an anti-PD-1 antibody include nivolumab, pembrolizumab, spartalizumab, and cemiplimab. Examples of an anti-PD-Ll antibody include atezolizumab, durvalumab, and avelumab. Examples of an anti-CTLA-4 antibody include ipilimumab and tremelimumab.

As used herein, an "immune checkpoint factor" is a factor that suppresses an autoimmune response in order to maintain homeostasis and also suppresses an excessive immune reaction. An "immune checkpoint factor" originally exists in order to suppress excessive activation of a T cell and not to attack itself. However, an immune checkpoint factor is utilized by a cancer cell to avoid an attack from the immune system and proliferate during the carcinogenesis process. Thus, inhibition of an immune checkpoint factor in a cancer cell enables a T cell to attack the cancer cell. Representative examples of an "immune checkpoint factor" include PD-1, PD-L1, PD-L2, CTLA-4, LAG-3, TIM-1, TIM-3, TIM-4, VISTA, BTLA, TIGIT, A2AR, 4-1BB, 4-1BBL, 2B4 (CD244), KIR family receptors, B7.1, B7.2, B7-H2, B7-H3, B7-H4, B7-H6, BATE, CD39, CD40, CD47, CD48, CD73, CD94/NKG2A, CD96, CD160, CD200, CD200R, CD274, butyrophilins, CEACAM1, CSF-1R, DcR3, EDO, Foxp1, GARP, GITR, gp49B, HHLA2, HVEM, ICOS, IDO, ILT-2, ILT-4, LAIR-1, MAFB, MICA./B, NKG2A/HLA-E, NR4A2, OCT-2, OX-40, PIR-B, Rara (retinoic acid receptor alpha), SIRP, TDO, TLR3, and TNFR.

As used herein, a "direct dendritic cell activating agent or means (device)" refers to an agent or means capable of directly (in other words, not via other molecules) activating a dendritic cell inside or outside the body of a subject. Representative examples of a "direct dendritic cell activating agent or means" include a radiation therapy providing means and an immunoadjuvant.

In the present disclosure, whether a certain substance "directly activates" a "dendritic cell" can be determined in the following manner: as exemplified in the Examples or the like, when a target substance or factor that is a candidate has higher expression of CD80/86 that is a cell membrane surface marker than that in the absence of the target substance or factor (e.g., saline) + the control antibody group, the target substance or factor that is a candidate is considered as directly activating the cell, and it can be determined that the target substance or factor falls under the "direct dendritic cell activating agent or means (device)".

As used herein, a "radiation therapy providing means" refers to a method, an apparatus, an equipment, an agent, a device and the like for providing radiation therapy to a subject.

As used herein, a "medical device" refers to an object that is inserted or transplanted into a target or applied to the surface of a target for treatment, prevention, or prevention of recurrence. In addition to any device that is used for radiation therapy, general examples of the medical device include stents, fasteners, ports, catheters, scaffolds, grafts, and the like.

As used herein, an "EGFR gene mutation" refers to a mutation in the EGFR gene. "EGFR" means epidermal growth factor receptor and functions in proliferation and growth of a cell. When a mutation occurs in the EGFR gene, proliferation and growth of a cell are constantly activated, which causes canceration. An "EGFR gene mutation" is known to cause non-squamous cell carcinoma, which is one type of lung cancer.

As used herein, a "tyrosine kinase inhibitor (TKI)" refers to an agent that inhibits tyrosine kinase. A tyrosine kinase inhibitor has an action of suppressing cancer proliferation by blocking a signaling pathway involved in proliferation of a cancer cell. Representative examples of a "tyrosine kinase inhibitor" include afatinib, erlotinib, osimertinib (AZD9291), AZD3759, gefitinib, canertinib, lapatinib, cetuximab, matuzumab, zalutumumab, and panitumumab.

As used herein, an "immunoadjuvant" refers to any agent or factor that assists an immune reaction. Representative examples thereof can include a *Mycobacterium tuberculosis* extract such as a human *Mycobacterium tuberculosis* hot water extract or a portion thereof.

As used herein, "CXCL10" is an abbreviation of C-X-C motif chemokine ligand 10 and is also known as IP-10, interferon gamma-induced protein 10, or Small-inducible cytokine B10. CXCL10 is an 8.7 kDa protein that, in humans, is encoded by the CXCL10 gene, is a non-glycosylated protein, and consists of 77 amino acids. CXCL10 is a chemokine that is produced in response to treatment of a monocyte, an endothelial cell, or a fibroblast with IFNγ. IP-10 functions as a chemotaxis inducer cell expressing a G protein-coupled receptor, CXCR3 that has been found mainly in an activated T cell or NK cell. In addition, IP-10 is also known as CXCL10, C7, IFI10, INP10, IP-10, SCYB10, crg-2, gIP-10, mob-1, C-X-C motif chemokine ligand 10, C-X-C motif chemokine 10, or the like. IP-10 is also known by its IDs, NM_001565 (nucleic acid) or NP_001556 (protein).

As used herein, "enhancement of CXCL10 production" refers to increasing the production (or amount of substance) of CXCL10.

As used herein, "cancer or tumor that is CXCL10 positive" refers to cancer or tumor being CXCL10 positive.

As used herein, "CXCR3" has the same meaning as the meaning commonly used in the art, and is one of CXC chemokine receptor families, which are G protein-coupled receptors. In addition to G protein-coupled receptor 9 (GPR9) and CD183, CXCR3 may be referred to as CD182; CKR-L2; CMKAR3; IP10-R; Mig-R; MigR, or the like. Two mutants of CXCR3 are known. While CXCR3-A, one of the mutants, binds to CXCL9 (MIG), CXCL10 (IP-10), and CXCL11 (I-TAC), which are CXC chemokines, CXCR3-B can further bind to CXCL4 in addition to those chemokines. While examples of the nucleic acid ID include NM_001142797, NM_001504, and the like, examples of the protein ID can include NP_001136269, NP_001495, and the like.

As used herein, a "CD8⁺ T cell^{low}" refers to a T cell positive for CD8 expression. As used herein, infiltration of CD8 positive T cells are evaluated in accordance with the cell count counted by confirming a slide using at least three different high magnification fields (40 power objective and 10 power eyepiece at the maximum). The number of cells stained as CD8 positive is recorded, and the case where the number of cells is 5 or less in three fields is defined as low infiltration, while the case where the number of cells is more than 5 is defined as high infiltration.

As used herein, a "human *Mycobacterium tuberculosis* hot water extract" is typically a substance produced from a human *Mycobacterium tuberculosis,* which is a mixture including polysaccharides with arabinose, mannose, and glucose as the primary ingredients. While anticancer effects due to human *Mycobacterium tuberculosis* hot water extracts have been studied for a long time, the detailed mechanism of action thereof is not necessarily elucidated. Further, the extract has not been used as a prophylactic drug. The extract can also comprise a trace amount of ingredients such as a protein, peptide, amino acid, nucleic acid, or lipid (glycolipid) when appropriate. Examples of a human *Mycobacterium tuberculosis* hot water extract can include Extract Z described herein or the like. Thus, the technology of the present disclosure can use Extract Z described in detail herein or any formulation manufactured using Extract Z as an active pharmaceutical ingredient.

The following is a representative manufacturing method of human *Mycobacterium tuberculosis* hot water extracts.

Human *Mycobacterium tuberculosis* is cultured for 3 to 7 weeks in a 37°C thermostatic vessel. The film of microbial cells formed on a medium is then filtered out. The moist microbial cells with medium components removed by washing with water are used as the extracted raw material. The microbial cells are floated in a distilled water at an amount that is 15 to 40-fold of the wet weight thereof, and heated for 80 to 180 minutes at 90 to 120°C for extraction. The residue of the microbial cells is removed with a sterilization filter, and the extracted solution is concentrated to 60% or less, and then acetone, trichloroacetate, ammonium sulfate, sulfosalicylic acid, or the like is added thereto so as to arrive at 0.5 to 3% (w/v), and stirred and left standing. The deposited precipitate is then centrifuged and removed, and the supernatant is subjected to running water dialysis. Inner fraction of dialysis fluid is subjected to vacuum concentration to a 1/20 to 1/4 volume, and sodium chloride is added to the concentrate so as to arrive at 0.5 to 1% (w/v). 2 to 4-fold volume of ethanol is added and left standing, and then the precipitate is removed by centrifugation. After adding an additional 2 to 6-fold volume of ethanol and incubating, a precipitating polysaccharide is obtained by centrifugation or the like. Those skilled in the art can understand that each of the conditions described above can be appropriately changed to obtain the same product.

As used herein, "prophylaxis" or "prevention" is an act of administering an active ingredient in the present disclosure to an individual who has not developed the target disease, intended to for example prevent development of the disease.

As used herein, "treatment" or "therapy" is, for example, an act of administering an active ingredient of the present disclosure to an individual (subject, patient) diagnosed as having a developed disease by a physician or a similar practitioner, intended to, for example, alleviate the disease or condition, not increase carcinoma, or revert back to the state before the development of the disease. Even if the objective of administration is prevention of exacerbation of the disease or condition or prevention of increase in the carcinoma, the administration is a therapeutic act if administered to a patient.

As used herein, "radiation therapy" refers to a therapeutic method by irradiation of a radiation. Examples of a radiation include X-rays, gamma rays, electron beams, proton beams, heavy particle beams, and the like. For a radiation, one type of radiation may be used, or two or more types of radiation may be used. In one aspect, examples of the "radiation therapy providing means" of the present disclosure can include a radiosensitizer, a radiation irradiating device, a radioactive substance, and the like. In the present disclosure, the "radiation therapy providing means" is preferably configured to provide palliative irradiation. More preferably, the "radiation therapy providing means" is configured to irradiate to have an abscopal effect. In still another embodiment, the "radiation therapy providing means" is configured to irradiate a measurable lesion that is present other than a lesion that is a target.

As used herein, a "regulatory T cell inhibitor" refers to any agent, device, or the like that suppresses regulatory T cells. Whether regulatory T cells is suppressed can be determined by, for example, the following approach:
(1) Collecting CD4⁺CD25⁺ cells from the spleen or tumor from a mouse and co-culturing the cells with effector T cells collected/differentiated from the spleen of a mouse to confirm proliferation of the effector T cells. Performing treatment with an agent by administering the agent in vivo or adding the agent in vitro when culturing the CD4⁺CD25⁺ cells.
(2) Transplanting cancer cells into a mouse and administering the agent to the mouse. Collecting tumor and calculating the ratio of FoxP3⁺ cells in CD4⁺CD25⁺ cells infiltrating into the tumor.
(3) Transplanting cancer cells into a mouse and administering the agent to the mouse. Collecting tumor and confirming FoxP3 gene expression.

The target animal is not limited to mice and may be other animals (which can include humans). Examples of regulatory T cells inhibitor can include, but are not limited to, a COX-2 inhibitor and the like.

As used herein, a "COX-2 inhibitor" refers to an inhibitor of prostaglandin-endoperoxide synthase 2. Examples can include celecoxib, etodolac, meloxicam, nabumetone, zaltoprofen, lornoxicam, and the like.

The term "carrier" as used herein refers to a pharmaceutically acceptable substance, composition, or excipient such as, for example, a liquid or solid bulking agent, diluent, additive, solvent, or capsule forming agent, which is associated with or enables the transport or carriage of a target pharmaceutical compound from an organ or portion of the body to another organ or portion of the body. "Pharmaceutically acceptable" refers to being compatible with other raw materials in a formulation and being harmless to patients. Non-limiting examples of pharmaceutically acceptable carriers, carriers, and/or diluents can include sugars such as lactose, glucose, and sucrose, starch such as corn starch and potato starch, cellulose and derivatives thereof such as carboxymethylcellulose sodium, ethyl cellulose, and cellulose acetate, excipients such as powdered tragacanth, malt, gelatin, talc, cocoa powder, and suppository wax, oil such as peanut oil, cotton seed oil, safflower oil, sesame oil, olive oil, corn oil, and soybean oil, glycols such as propylene glycol, polyols such as glycerin, sorbitol, mannitol, and polyethylene glycol, esters such as ethyl oleate and ethyl laureate, buffering agents such as agar, magnesium hydroxide, and aluminum hydroxide, alginic acid, pyogenic substance-free water, isotonic saline, Ringer's solution, ethyl alcohol, phosphate buffer, and other nontoxic compatible substances used in a pharmaceutical formulation. A humectant, emulsifier, and lubricant such as sodium lauryl sulfate, magnesium stearate, or polyethylene oxide-polypropylene oxide copolymer, as well as a colorant, releasing agent, coating agent, sweetener, flavoring agent, fragrance, preservative, and antioxidant can also be included in a composition.

As used herein, "parenteral administration" refers to a dosage form for any route that is not oral administration. Any mode for administration in a mode and level that are effective for treating or preventing a disease intended for cancer treatment or prevention is employed. Examples of means of parenteral administration include administration through transdermal absorption or transmucosal absorption, as well as injection, infusion, and combinations thereof. For example, administration through transdermal absorption or transmucosal absorption exerts an effect by contacting a transdermally absorbed formulation such as a paste agent, adhesive formulation, or spray with the skin or mucous membrane so that a drug in the formulation migrates into the body through the skin or mucous membrane. Examples of administration via injection or infusion include intravenous, intradermal, subcutaneous, intramuscular, and enteral administration (intestinal infusion), which can also be administered as a bolus and/or sustained infusion. Injection or infusion can use a suspension, liquid agent, emulsion, or implanted agent in an oily or aqueous medium, comprising another formulation substance such as a suspending agent, stabilizer, and/or a dispersant. Enteral administration (intestinal infusion) can provide sustained drug delivery to the proximal small intestine by using a tube or portable infusion pump by percutaneous endoscopic gastrostomy. More preferably, the administration can be subcutaneous or intradermal administration. Parenteral administration (e.g., transdermal administration) can be performed with a tape/patch agent or powder, spray, ointment, paste, cream, lotion, gel, solution, or the like. A composition suitable for parenteral administration can comprise at least one type of a pharmaceutically acceptable aseptic isotonic aqueous or non-aqueous solution, dispersant, suspension, emulsion, implanted agent, or aseptic powder that can be reconstituted in an aseptic injection solution or dispersant immediately before use.

### (Description of preferred embodiments)

The preferred embodiments of the present disclosure are described hereinafter. It is understood that the embodiments provided hereinafter are provided to better facilitate the understanding of the present disclosure, and thus the scope of the present disclosure should not be limited by the following descriptions. Thus, it is apparent that those skilled in the art can refer to the descriptions herein to make appropriate modifications within the scope of the present disclosure. It is also understood that the following embodiments of the present disclosure can be used alone or in combination.

### <Direct dendritic cell activation>

In one aspect, the present disclosure provides various agents or means for directly activating a dendritic cell. In one embodiment, the present disclosure provides a composition or use for directly activating a dendritic cell which comprises an immunoadjuvant, and a method using the composition or use. In an exemplary embodiment, an immunoadjuvant comprises a *Mycobacterium tuberculosis* extract such as a human *Mycobacterium tuberculosis* hot water extract or a portion thereof.

In another embodiment, the present disclosure provides a composition or medical device or use for activating a dendritic cell which comprises a radiation therapy providing means, and a method using the composition or medical device or use. In this case, the radiation therapy providing means comprises at least one selected from the group consisting of a radiosensitizer, a radiation irradiating device, and a radioactive substance. For example, a radiation therapy providing means is configured to provide palliative irradiation. In a preferred embodiment, a radiation therapy providing means is configured to irradiate to have an abscopal effect. In a specific example, a radiation therapy providing means is configured to irradiate a measurable lesion that is present other than a lesion that is a target.

In the present disclosure, whether a dendritic cell is directly activated can be determined by confirming an increase in the presence of a target substance or factor compared to in the absence of the target substance or factor in an experiment comprising measuring an increase in the amount of expression of CD80/86 on the surface of the dendritic cell in the presence of an immunoadjuvant compared to in the absence of the immunoadjuvant.

### <Combined use of immune checkpoint inhibition and direct dendritic cell activation>

The present disclosure is based on the discovery that combined use of an immune checkpoint inhibitor and a direct dendritic cell activating agent or means attains a therapeutic and preventive effect, which is specific and effective against neoplasm such as cancer.

The present disclosure generally provides a combination for treatment, prevention, or prevention of recurrence of cancer or tumor of a subject by combined use of an immune checkpoint inhibitor and a direct dendritic cell activating agent or means, a method for the treatment, prevention, or prevention of recurrence, and a combination of an immune checkpoint inhibitor and a direct dendritic cell activating agent or means for use in the treatment, prevention, or prevention of recurrence. The immune checkpoint inhibitor and the direct dendritic cell activating agent or means may be administered or provided at the same time or at different times. Any of administration of the immune checkpoint inhibitor and administration of the direct dendritic cell activating agent or means may precede.

In another aspect, the present disclosure provides a composition, use or a medical device, use, a therapeutic method, a preventive method, or a recurrence preventing method for treatment, prevention, or prevention of recurrence of cancer or tumor of a subject, using a direct dendritic cell activating agent or means. The direct dendritic cell activating agent or means is administered or used in combination with an immune checkpoint inhibitor.

In still another aspect, the present disclosure provides a composition, use or a medical device, use, a therapeutic method, a preventive method, or a recurrence preventing method for treatment, prevention, or prevention of recurrence of cancer or tumor of a subject, using an immune checkpoint inhibitor. The immune checkpoint inhibitor is administered or used in combination with a direct dendritic cell activating agent or means.

In one representative aspect, examples of a direct dendritic cell activating agent or means can include an immunoadjuvant, a radiation therapy providing means, a radioactive substance, a combination thereof, and the like.

The immune checkpoint inhibitor or the direct dendritic cell activating agent or means of the present disclosure can be provided in a dosage form of a pharmaceutical composition. In a specific embodiment, a pharmaceutical composition can comprise one or more types of compounds and at least one type of pharmaceutically acceptable carrier, wherein the one or more types of compounds can be converted into, for example, at least one type of compound of a *Mycobacterium tuberculosis* extract in a subject (in other words, the one or more types of compounds may be provided as a prodrug).

In one embodiment, examples of an immunoadjuvant that can be used in the present disclosure can include a human *Mycobacterium tuberculosis* hot water extract or a portion thereof, and the like.

In one embodiment, any means (such as a device) that provides an X-ray, a gamma ray, an electron beam, a proton beam, a heavy particle beam, or the like can be used as a radiation therapy providing means, wherein one type of radiation may be used, or two or more types of radiation may be used as a radiation. In one aspect, the radiation therapy providing means of the present disclosure may comprise a radiosensitizer, may be used with a radiation irradiating device, may be provided with a radioactive substance, or may comprise any combination thereof. In the present disclosure, a radiation therapy providing means is preferably configured to provide palliative irradiation. More preferably, a radiation therapy providing means is configured to irradiate to have an abscopal effect. Furthermore, in one embodiment, a radiation therapy providing means is configured to irradiate a measurable lesion that is present other than a lesion that is a target.

In the present disclosure, whether a dendritic cell is directly activated can be determined by confirming an increase in the presence of a target substance or factor compared to in the absence of the target substance or factor in an experiment comprising measuring an increase in the amount of expression of CD80/86 on the surface of the dendritic cell in the presence of an immunoadjuvant compared to in the absence of the immunoadjuvant.

In one preferred embodiment, an immune checkpoint inhibitor can be an inhibitor of a factor such as PD-1, PD-L1, or CTLA-4. An immune checkpoint inhibitor may be a combination of inhibitors of these factors. In one example, an immune checkpoint inhibitor may be a PD-1 inhibitor, a PD-L1 inhibitor, a CTLA-4 inhibitor, or the like. One agent may be an inhibitor of a plurality of factors, PD-1, PD-L1, and CTLA-4. One specific example can be, but is not limited to, nivolumab, pembrolizumab, atezolizumab, or the like.

In one embodiment, it is preferable, but is not limited to, that cancer expresses PD-L1. In another embodiment, cancer can include, but is not limited to, lung cancer. In a preferred embodiment, cancer or tumor can be, but is not limited to, cancer or tumor having an EGFR gene mutation. Thus, in one example, treatment, prevention, or prevention of recurrence can be for a subject who has received therapy with a tyrosine kinase inhibitor (e.g., EGFR-TKI). In one specific embodiment, therapy, prevention, or prevention of recurrence can be performed on a subject who has not received therapy with an immune checkpoint inhibitor. In another embodiment, treatment, prevention, or prevention of recurrence can be performed on a subject for whom therapy with an immune checkpoint inhibitor was determined to be ineffective. In one specific embodiment, treatment, prevention, or prevention of recurrence of the present disclosure can be performed on, but not limited to, a subject who received therapy with an immune checkpoint inhibitor which was effective, but subsequently experienced progressive disease (PD).

In one embodiment, a subject is a subject who is CXCL10 positive in the subject's tumor because a CXCR3 positive cell can easily invade. In another embodiment, the technology of the present disclosure is used for enhancement of CXCL10 production. In another embodiment, a subject is a CD8⁺ T cell^{low} subject. Thus, the technology of the present disclosure can be used to promote infiltration of CD8⁺ T cells into tumor. In a specific embodiment, the present disclosure can be used for treatment, prevention, or prevention of recurrence of Tumor Mutational Burden^{high} and CD8⁺ T cell^{low} cancer that is inoperable or metastatic solid cancer for which there is no other therapeutic choice.

In an embodiment of the present disclosure, the present disclosure may be used with a regulatory T cell inhibitor. A COX-2 inhibitor can be used as a regulatory T cell inhibitor. Preferably, a COX-2 inhibitor can be used as a regulatory T cell inhibitor. Examples that can be used as a COX-2 inhibitor include, but are not limited to, celecoxib, other COX-2 inhibitors (e.g., etodolac, meloxicam, nabumetone, zaltoprofen, and lornoxicam), and the like.

### (Medical technology such as medicaments or therapy)

A medicament for use in the technology for treatment, prevention, or prevention of recurrence of cancer of the present disclosure can be used by any approach that is known as a pharmaceutical product in the art.

In a specific embodiment, a pharmaceutical composition can comprise one or more types of compounds and at least one type of pharmaceutically acceptable carrier, wherein the one or more types of compounds can be converted into at least one type of *Mycobacterium tuberculosis* extract (i.e., prodrug) in a subject. A plurality of agents, when included, can be included in a single composition (combined agent) or in separate compositions. The agents can be formulated as a single composition using a known embodiment in the art, including those exemplified herein. A plurality of agents may be provided to achieve a therapeutic method (e.g., anticancer agent administration, radiation therapy, or the like) or with one or more other medicaments (e.g., surgery or an anticancer agent such as a chemotherapeutic agent) in addition to the immune checkpoint inhibitor and/or direct dendritic cell activating agent or means of the present disclosure. The immune checkpoint inhibitor and/or direct dendritic cell activating agent or means of the present disclosure can be provided or administered in combination with one or more other medicaments or therapeutic methods (e.g., surgery, chemotherapeutic agent, radiation therapy, or anticancer agent). In one embodiment, one or more other medicaments or therapeutic methods (e.g., surgery, chemotherapeutic agent, radiation therapy, or anticancer agent) may be administered after an appropriate period has elapsed from administration of the immune checkpoint inhibitor and/or direct dendritic cell activating agent or means of the present disclosure. When administered separately, two or more medicaments may be provided as a kit. Non-limiting examples of anticancer agents include chemotherapeutic agents such as antimetabolites and alkylating agents, growth inhibitors, cytotoxic agents, agents used in radiation therapy, antiangiogenesis agents, apoptosis agents, anti-tubulin agents, anticancer antibiotics, anti-microtubule drugs, tyrosine kinase inhibitors, proteasome inhibitors, anaplastic lymphoma kinase inhibitors, Janus kinase inhibitors, CDK inhibitors, MEK inhibitors, Raf kinase inhibitors, PARP inhibitors, antibody drugs, other molecularly targeted drugs, platinum formulations, immunotherapy such as dendritic cell therapy, gene therapy, other low molecule drugs, other agents for treating cancer, and the like.

The compositions disclosed herein that are suitable for oral administration can be in a dosage form of a capsule, cachet, pill, tablet, lozenge (generally using a fragrance base tragranth or acacia and sucrose), powder, granule, aqueous or non-aqueous liquid solution, aqueous or non-aqueous liquid suspension, oil-in-water emulsion, water-in-oil emulsion, elixir, syrup, troche (using inactive base such as gelatin, glycerin, sucrose, and/or acacia), and/or mouthwash, which each comprises a predetermined amount of at least one type of compound in the present disclosure.

A composition disclosed herein can be administered as a bolus, an electuary or paste.

The immune checkpoint inhibitor and/or direct dendritic cell activating agent or means of the present disclosure can be administered in any dosage form. Any dosage form, whether oral administration or parenteral administration, can be used, as long as the effect can be exerted. Preferably, parenteral administration is used.

A solid dosage form for oral administration (capsule, tablet, pill, sugar coated tablet, powder, granule, or the like) can be mixed with any of one or more types of pharmaceutically acceptable carrier such as sodium citrate or dicalcium phosphate, and/or a filler or bulking agent such as starch, lactose, sucrose, glucose, mannitol, and/or silisic acid, binding agent such as carboxymethyl cellulose, alginic acid salt, gelatin, polyvinyl pyrrolidone, sucrose, and/or acacia, a moisturizing agent such glycerol, a disintegrant such as agar, calcium carbonate, potato or tapioca starch, alginic acid, specific silicic acid salt, sodium carbonate, and sodium starch glycolate, a dissolution delaying agent such as paraffin, an absorption promotor such as a quaternary ammonium compound, humectant such as cetyl alcohol, glycerol monostearate, and polyethylene oxide-polypropylene oxide copolymer, an absorbent such as kaolin and bentonite clay, a lubricant such as talc, calcium stearate, magnesium stearate, solid polyethylene glycol, sodium lauryl sulfate, and mixture thereof, and a colorant. For a capsule, tablet, and pill, a pharmaceutical composition can also comprise a buffering agent. A similar type of solid composition can also be used as a filler in a soft and hard filled gelatin capsule using an additive such as lactose and high molecular weight polyethylene glycol.

A liquid dosage form for oral administration can comprise a pharmaceutically acceptable emulsion, microemulsion, solution, suspension, syrup, and elixir. In addition to the active ingredient, a liquid dosage form can comprise an inactive diluent used in conventional art, such as water or other solvent, solubilizing agent, and emulsifying agent, e.g., ethyl alcohol, isopropyl alcohol, ethyl carbonate, ethyl acetate, benzyl alcohol, benzyl benzoate, propylene glycol, 1,3-butylene glycol, oil (especially cotton seed oil, peanut oil, corn oil, germ oil, olive oil, castor oil, and sesame oil), glycerol, tetrahydrofuryl alcohol, polyethylene glycol, sorbitan fatty acid ester, mixture thereof, and the like. Furthermore, a compound can be dissolved using cyclodextrin such as hydroxypropyl-β-cyclodextrin.

The component of the present disclosure can comprise an adjuvant such as a humectant, emulsifying and suspending agent, sweetener, flavoring agent, colorant, fragrance, or preservative. In addition to one or more types of compounds according to the present disclosure, a suspension can comprise a suspending agent such as ethoxylated isostearyl alcohol, polyoxyethylene sorbitol and sorbitan ester, microcrystalline cellulose, aluminum methhydroxide, bentonite, agar, tragacanth, or mixture thereof.

The combination disclosed herein can be a suppository for rectal or vaginal administration, which can be prepared by mixing one or more types of compounds according to the present disclosure with one or more types of suitable non-stimulatory additives or carriers including cocoa butter, polyethylene glycol, suppository wax, salicylate, or the like. The composition is a solid at room temperature, but a liquid at body temperature. Thus, the composition melts in the rectal or vaginal cavity and releases the compound of the present disclosure. A pharmaceutical composition suitable for vaginal administration can also comprise a pessary, tampon, cream, gel, paste, foam, or spray formulation comprising a carrier known to be suitable in conventional art.

The dosage form for topical or transdermal administration of the combination of the present disclosure can comprise powder, spray, ointment, paste, cream, lotion, gel, solution, patch, and inhalant. A pharmaceutical composition or pharmaceutical tablet can be mixed with a pharmaceutically acceptable carrier and a preservative, buffering agent, or high pressure gas that may be required under aseptic conditions.

An ointment, paste, cream, and gel can comprise, in addition to the combination of the present disclosure, an additive such as animal or vegetable fat, oil, wax, paraffin, starch, tragacanth, cellulose derivative, polyethylene glycol, silicone, bentonite, silicic acid, talc, zinc oxide, or mixture thereof.

Powder or spray can comprise, in addition to the pharmaceutical composition or pharmaceutical tablet of the present disclosure, an additive such as lactose, talc, silicic acid, aluminum hydroxide, calcium silicate, or polyamide powder, or a mixture thereof. Furthermore, spray can comprise common high pressure gas such as chlorofluorohydrocarbon and volatile unsubstituted hydrocarbon such as butane and propane.

Ophthalmic formulations, optical ointment, powder, solution, and the like are also understood to be within the scope of the present disclosure.

A composition suitable for parenteral administration can comprise at least one type of pharmaceutically acceptable aseptic isotonic aqueous or non-aqueous solution, dispersant, suspension, emulsion, or aseptic powder that can be reconstituted in an aseptic injection solution or dispersant immediately before use.

The term "salt" as used herein includes acid and/or base salt formed with inorganic and/or organic acid and base. As used herein, the term "pharmaceutically acceptable salt" refers to a salt which is suitable for use in contact with tissue of a subject without excessive toxicity, stimulation, allergic reaction, and/or similar events with a reasonable balance of effect/risk ratio within the scope of a definitive medical judgment. Pharmaceutically acceptable salts are well known in the art. For example, pharmaceutically acceptable salts are described in detail in Berge et al., J. Pharmaceutical Sciences (1977) 66: 1-19.

Pharmaceutically acceptable salts can be produced with an inorganic or organic acid. Non-limiting examples of suitable inorganic salts include hydrochloric acid, hydrobromic acid, phosphoric acid, sulfuric acid, and perchloric acid. Non-limiting examples of suitable organic salts include acetic acid, oxalic acid, maleic acid, tartaric acid, citric acid, succinic acid, and malonic acid. Other non-limiting examples of suitable pharmaceutically acceptable salts include adipic acid salt, alginic acid salt, ascorbic acid salt, aspartic acid salt, benzenesulfonic acid salt, besilate, benzoic acid salt, bisulfuric acid salt, boric acid salt, butyric acid salt, camphoric acid salt, camphorsulfonic acid salt, citric acid salt, cyclopentanepropionic acid salt, digluconic acid salt, dodecylsulfuric acid salt, ethanesulfonic acid salt, formic acid salt, fumaric acid salt, glucoheptonic acid salt, glycerophosphoric acid salt, gluconic acid salt, hemisulfuric acid salt, heptanoic acid salt, hexanoic acid salt, hydroiodic acid salt, 2-hydroxy-ethanesulfonic acid salt, lactobionic acid salt, lactic acid salt, lauric acid, lauryl sulfate, malic acid salt, maleic acid salt, malonic acid salt, methanesulfonic acid salt, 2-naphthalenesulfonic acid salt, nicotinic acid salt, nitric acid salt, oleic acid salt, oxalic acid salt, palmitic acid salt, pamoic acid salt, pectinic acid salt, persulfuric acid salt, 3-phenylpropionic acid salt, phosphoric acid salt, picric acid salt, pivalic acid salt, propionic acid salt, stearic acid salt, succinic acid salt, sulfuric acid salt, tartaric acid salt, thiocyanic acid salt, p-toluenesulfonic acid salt, undecanoic acid salt, and valeric acid salt. In some embodiments, examples of organic acids that can produce a salt include acetic acid, propionic acid, glycolic acid, pyruvic acid, oxalic acid, lactic acid, trifluoroacetic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, and salicylic acid.

A salt can be prepared at the time of separation and purification of a disclosed compound or separately by reacting the compound with a suitable base or acid. Non-limiting examples of pharmaceutically acceptable salts obtained from a base include alkali metals, alkali earth metals, ammonium, and N+(C1-4 alkyl)4 salts. Non-limiting examples of suitable alkali or alkali earth metal salts include sodium, lithium, potassium, calcium, magnesium, iron, zinc, copper, manganese, and aluminum salt. Furthermore, non-limiting examples of suitable pharmaceutically acceptable salts optionally include nontoxic ammonium, quaternary ammonium, and amine cation formed using a counter ion such as a halide ion, hydroxide ion, carboxylic acid ion, sulfuric acid ion, phosphoric acid ion, nitric acid ion, lower alkyl sulfonic acid ion, and aryl sulfonic acid ion. Non-limiting examples of suitable organic bases that can produce a salt include primary amine, secondary amine, tertiary amine, substituted amine including naturally-occurring substituted amine, cyclic amine, isopropylamine, trimethylamine, diethylamine, triethylamine, tripropylamine, ethanol amine, and other basic ion exchange resins. In a specific embodiment, a pharmaceutically acceptable base addition salt can be selected from ammonium, potassium, sodium, calcium, and magnesium salt.

In an embodiment of the present disclosure, a target subject can be a patient in a state before onset of cancer, after a cancer treatment, an early stage of onset of cancer, or a precancerous condition. Alternatively, a target subject can be a healthy individual. If a healthy individual is a subject, the disclosure is performed as a preventive method.

Examples of cancer targeted in the present disclosure include, but are not limited to esophageal cancer, gastroesophageal junction cancer, renal cell cancer, lung cancer, digestive organ cancer, leukemia, lymphoma, myeloma, brain cancer, pancreatic cancer, endometrial cancer, prostate cancer, liver cancer, bladder cancer, gastroesophageal adenocarcinoma, chondrosarcoma, colorectal adenocarcinoma, colorectal cancer, breast cancer, renal cell cancer, ovarian cancer, head and neck cancer, melanoma, gastric adenocarcinoma, sarcoma, urogenital cancer, gynecological cancer, adrenocortical cancer, and the like. In a specific embodiment, cancer is lung cancer. In a specific embodiment, cancer is colorectal cancer. In a specific embodiment, cancer is colorectal adenocarcinoma. In a specific embodiment, cancer is melanoma. In a specific embodiment, cancer is breast cancer. In a specific embodiment, cancer is bladder cancer. In a specific embodiment, cancer is renal cell cancer. In a specific embodiment, cancer is pancreatic cancer. In a specific embodiment, cancer is endometrial cancer. In a specific embodiment, cancer can be unresectable. In a specific embodiment, cancer can be progressive. In a specific embodiment, cancer can be refractory. In a specific embodiment, cancer can be recurrent. In a specific embodiment, cancer can be metastatic. In various embodiments of the present disclosure, target cancer can include normal carcinoma, carcinoma with a relatively slow progression (e.g., cancer with low sensitivity to the immune system), oral squamous cell cancer, cervical cancer, MHC class I negative carcinoma on which CD8 positive T cells are generally less effective, immune checkpoint inhibitor resistant cancer, and the like. A cancer patient refers to a patient suffering from a "cancer" described above. In one embodiment, the target disease, disorder, or condition of the present disclosure comprises melanoma.

Although not wishing to be bound by any theory, a human T cell has a portion that can bind to a factor of an autologous cell in order not to attack a cell other than a T cell of an individual. The representative factor (molecule) of the portion is PD-1. Meanwhile, a general cell has a factor (molecule) such as PD-L1. For example, PD-L1 binds to PD-1, whereby a general cell escapes an attack from a T cell (immune cell). This mechanism is an immune checkpoint system as a system suppressing the activity of a T cell. Since a cancer cell itself is also an autologous cell, a cancer cell escapes an attack from a T cell (immune cell) by the immune checkpoint system.

Although not wishing to be bound by any theory, an immune checkpoint inhibitor (ICI) is, for example, an agent that inhibits the immune checkpoint system by inhibiting a stimulus to an immune checkpoint molecule (PD-1), which is an immune function inhibitory receptor present in a T cell that is an immune cell. Since an immune checkpoint inhibitor is an agent that inhibits a system suppressing the immunity, administration of the agent activates the immune function of a patient. In addition, inhibition of an immune checkpoint molecule in a cancer cell enables a T cell to attack the cancer cell.

Although not wishing to be bound by any theory, Extract Z is a suitable therapeutic agent that is used to increase sensitivity to an immune checkpoint inhibitor. Extract Z was developed as a therapeutic drug of tuberculosis, is a colorless and transparent subcutaneous injection solution, and comprises, as primary ingredients, a polysaccharide called Lipoarabinomannan extracted from human *Mycobacterium tuberculosis,* a nucleic acid, and a lipid.

Although not wishing to be bound by any theory, administration of Extract Z attains an effect of increasing sensitivity to an immune checkpoint inhibitor (ICI). Apart from administration of these therapeutic agents, radiation irradiation can also be expected to attain an effect of increasing sensitivity to an immune checkpoint inhibitor (ICI) .

In one embodiment, therapy with an immune checkpoint inhibitor (first therapy) and therapy for increasing sensitivity to an immune checkpoint inhibitor (second therapy) as described above are combined to treat cancer of a patient. In other words, the method for cancer treatment of the present embodiment comprises a step of performing therapy with administration of an immune checkpoint inhibitor (first therapy) on a cancer patient and a step of performing therapy for increasing sensitivity to the immune checkpoint inhibitor (second therapy) on the cancer patient.

In one embodiment, nivolumab, pembrolizumab, or atezolizumab, which is an antibody against PD-1 of a T cell, is used as an immune checkpoint inhibitor in first therapy, thereby inhibiting an immune checkpoint of PD-1 of a T cell and PD-L1 of a cancer cell. Radiation irradiation, administration of Extract Z, or a combination thereof is performed as therapy in second therapy.

In one embodiment, first therapy and second therapy may be combined in an embodiment wherein these first therapy and second therapy are performed on a patient at the same time for a predetermined period. First therapy and second therapy may also be combined in an embodiment wherein either one of the first therapy and the second therapy is performed on a patient for a predetermined period, and the other therapy is then performed on the patient for a predetermined period. First therapy and second therapy may also be combined in an embodiment wherein either one of the first therapy and the second therapy is performed on a patient for a predetermined period, and both of them are then performed on the patient for a predetermined period. First therapy and second therapy may also be combined in an embodiment wherein both of the first therapy and the second therapy are performed on a patient for a predetermined period, and only either one of them is then performed on the patient for a predetermined period. Furthermore, the embodiments as described above may be appropriately combined and periodically repeated.

In various embodiments described above, the timing at which therapy is changed is identified based on a predetermined change in the test result with respect to a patient (such as a medical image of the patient, a measurement result (measurement value) of a tumor marker of the patient, or a PET test result of the patient) as described below.

In one embodiment, since first therapy and second therapy are combined, it is possible to eliminate or reduce cancer tissue by the synergistic action of the effect of the first therapy and the effect of the second therapy. Although not wishing to be bound by any theory, cancer tissue of a patient can be eliminated or reduced by enabling a T cell to exert its cytotoxic power on a cancer cell by the first therapy (administration of an immune checkpoint inhibitor) while increasing sensitivity to the immune checkpoint inhibitor by the second therapy (radiation irradiation, administration of Extract Z, or combined use of administration of Extract Z + radiation irradiation).

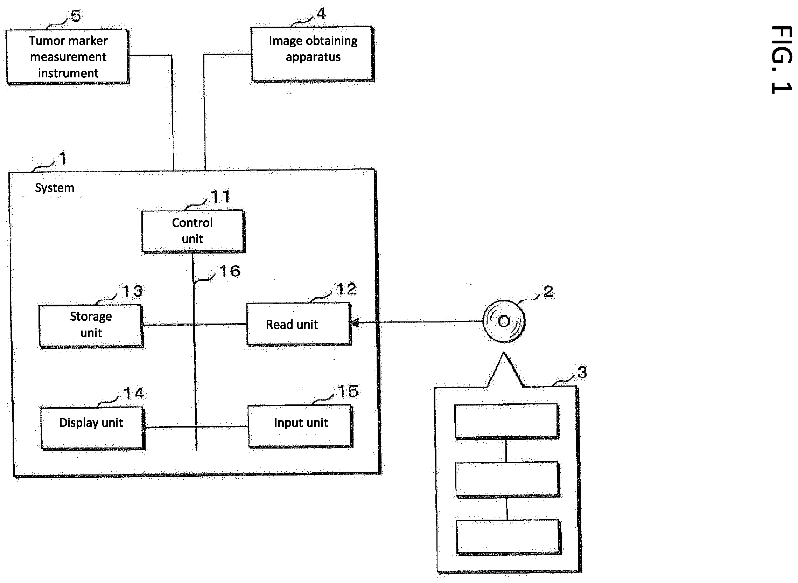

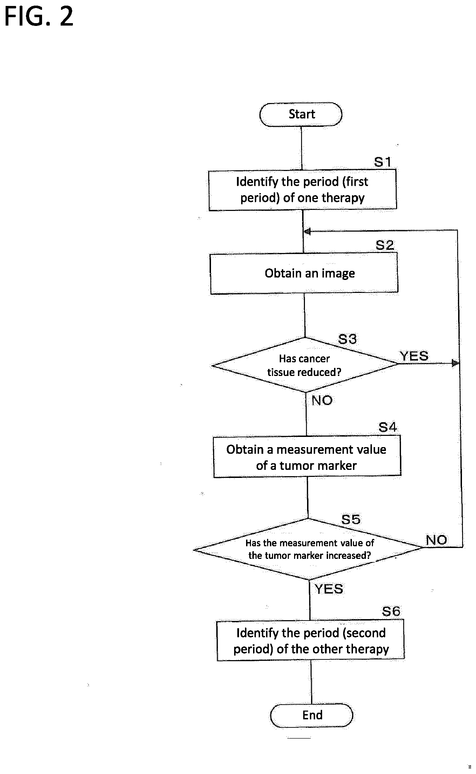

Figure 1 is a block diagram showing the configuration of a system that utilizes a program in the present embodiment. Figure 2 is a flowchart showing the therapy procedure in the present embodiment.

A system 1 shown in Figure 1 comprises a control unit 11, a read unit 12, a storage unit 13, a display unit 14, an input unit 15, and a bus 16. The control unit 11 is connected with other hardware units constituting the system 1 via the bus 16.

The control unit 11 is configured by using a CPU (Central Processing Unit), an MPU (Micro Processing Unit), or the like and executes a program in the present embodiment. The read unit 12 reads out a program 3 in the present embodiment which is recorded in a portable recording medium 2 such as flexible discs from the portable recording medium. The storage unit 13 is composed of an SRAM (Static Random Access Memory), a DRAM (Dynamic Random Access Memory), or the like and stores information necessary during processing performed by the control unit 11 and the program 3 read out by the read unit 12.

The display unit 14 is composed of a liquid crystal display panel or the like and displays history information of a patient, a result of processing by the control unit 11 (type of treatment performed on a patient, period during which each treatment is performed) or the like. The input unit 15 is composed of a keyboard, a mouse, or the like and receives input information from a user.

An image obtaining apparatus 4 for obtaining a medical image (such as CT images, X images, or PET images) of a patient is connected to the system 1 so that the medical image of the patient is input to the system 1 (control unit 11) regularly or in response to a request from the system 1 (control unit 11). A tumor marker measurement instrument 5 for measuring a tumor marker (specifically, CEA: Carcinoembryonic Antigen) of a patient is also connected to the system 1 so that a measurement value of a tumor marker (CEA) of the patient is input to the system 1 (control unit 11) regularly or in response to a request from the system 1 (control unit 11).

Although the program 3 is read out from the portable recording medium 2 by using read unit 12 in the example described above, the program 3 may be stored in the storage unit 13 in advance. Alternatively, the program 3 may be obtained from an external server via a network not illustrated.

Next, the treatment procedure in the present embodiment is described while referring to the flowchart of Figure 1.

The control unit 11 identifies a first period during which either one of therapy with an immune checkpoint inhibitor (first therapy) and therapy for increasing sensitivity to the immune checkpoint inhibitor (second therapy) is performed (step S1). In this step, it is also identified which of radiation irradiation, administration of Extract Z, and combined use of administration of Extract Z + radiation irradiation is performed as the second therapy. In this case, for example, the first period and the type of the second therapy are identified depending on an input from a user received via the input unit 15. The control unit 11 itself may identify the first period and the type of the second therapy based on the medical history, therapy history information in other cases of a patient, or the like. The identified first period and type of the second therapy are displayed on the display unit 14.

While continuing either one of the therapies, the control unit 11 obtains a medical image of the patient obtained at the image obtaining apparatus 4 (step S2).

The control unit 11 determines whether cancer tissue has reduced based on the obtained medical image (step S3). When cancer tissue has reduced (step S3: YES), the processing is returned to step S2.

Meanwhile, when cancer tissue has not reduced (step S3: NO), the control unit 11 obtains a measurement value of a tumor marker in the patient obtained by the tumor marker measurement instrument 5 (step S4).

The control unit 11 determines whether the obtained measurement value of the tumor marker has increased (step S5). If the measurement value has not increased (step S5: NO), the processing is returned to step S2.

Meanwhile, when the measurement value of the tumor marker has increased (step S5: YES), the control unit 11 identifies a second period during which the other of the therapy with an immune checkpoint inhibitor (first therapy) or the therapy for increasing sensitivity to the immune checkpoint inhibitor (second therapy) is performed (step S6), and the processing is then terminated. In this case, for example, the second period is identified depending on an input from a user received via the input unit 15. The control unit 11 itself may identify the second period based on the medical history, therapy history information in other cases of the patient, or the like. The identified second period is displayed on the display unit 14.

Although the period during which the other of the first therapy and the second therapy is performed is identified in step S6 in the embodiment described above, the period during which each of the first therapy and the second therapy is performed may be identified.

Although the period during which the other (or both) of the therapies is performed is identified when the measurement value of the tumor marker has increased in step S5 in the embodiment described above, the period during which the other (or both) of the therapies is performed may be identified when incorporation of glucose has increased based on the PET test result. It may be determined in step S5 whether the processing proceeds to the processing of step S6 (identifying the period during which the other (or both) of the therapies is performed) depending on both the variation in the measurement value of the tumor marker and the variation in incorporation of glucose based on the PET test result.

As used herein, "or" is used when "at least one or more" of the listed matters in the sentence can be employed. When explicitly described herein as "within the range of two values", the range also includes the two values themselves.

Reference literatures such as scientific literatures, patents, and patent applications cited herein are incorporated herein by reference to the same extent that the entirety of each document is specifically described.

As described above, the present disclosure has been described while showing preferred embodiments to facilitate understanding. The present disclosure described hereinafter is based on the Examples. The above descriptions and the following Examples are not provided to limit the present disclosure, but for the sole purpose of exemplification. Thus, the scope of the present disclosure is not limited to the embodiments and Examples specifically described herein and is limited only by the scope of claims.

### [Examples]

The Examples are described hereinafter. When necessary, animals used in the following Examples were handled in compliance with relevant ethical standards and guidelines, based on the Declaration of Helsinki. While the specific products described in the Examples were used, reagents can be substituted with an equivalent product from another manufacturer (Sigma-Aldrich, Wako Pure Chemical, Nacalai Tesque, R & D Systems, USCN Life Science INC, or the like).

### (Manufacturing Example: Extract Z)

Extract Z used in this Example was manufactured in the following manner.

Human *Mycobacterium tuberculosis* strain Aoyama B which had been lyophilized and stored (-20°C) was subjected to seed culture at 37 ± 1°C in a Sauton potato medium⁽¹⁾. The cultured bacteria were transferred to a production medium⁽²⁾ and cultured (primary culture) for 5 to 7 weeks at 37 ± 1°C. The resulting cells were washed with water for injection. To the cells, water for injection was then added in an amount 20-fold of the weight of the wet cells. The mixture was heated at 100°C for 120 minutes to obtain an extract. The extract was filtered with a 0.45 µm-membrane filter and then concentrated under reduced pressure so that the saccharide content (in terms of D-arabinose by the phenol-sulfuric acid method) would be 4.0 to 6.0 mg/ml to obtain a concentrate. Subsequently, in order to remove proteins, 1 w/v% of sulfosalicylic acid was added to the concentrate. The mixture was left standing for 15 to 20 minutes at 10°C or lower. Precipitates were then removed by centrifugation (10°C or lower, 1,150 x G, 10 minutes) to recover the supernatant. The protein concentration of the supernatant was 0.30 mg/ml or lower (Lowry method, in terms of tyrosine). The supernatant was further processed to remove sulfosalicylic acid until the concentration was at or below the detection limit (10 ppm or less, method using ferric chloride solution). The resultant solution was concentrated under reduced pressure so that the saccharide content would be 1.8 to 2.2 mg/ml, and the concentrate was combined with sodium chloride (0.9 w/v%) and cold ethanol at the same volume as the concentrate. The mixture was left standing for 40 hours or longer at 10°C or lower, and then the precipitates (polysaccharide of high molecular weight region) were removed by centrifugation (10°C or lower, 2,040 x G, 10 minutes). Subsequently, the supernatant was combined with four times the amount of cold ethanol, and the mixture was left standing for 40 hours or longer at 10°C or lower and centrifuged (10°C or lower, 2,040 × G, 10 minutes) to recover precipitates. The precipitates were dissolved in water for injection. After the saccharide content was adjusted to 1.8 to 2.2 mg/ml, the solution was filtered with a 0.45 µm membrane filter and sterilized with high pressure steam (121°C, 20 minutes) to prepare an Extract Z solution.

### (1) Sauton-potato medium

Washed potato slices were soaked in a Sauton medium, sterilized for 15 minutes at 115°C, and then used as a Sauton-potato medium.

### Sauton Medium

L-asparagine (monohydrate) 4.0 g
Citric acid (monohydrate) 2.0 g
Magnesium sulfate (heptahydrate) 0.5 g
Potassium monohydrogenphosphate (anhydride) 0.5 g
Ammonium iron citrate 0.05 g
Glycerol 60 ml

The above ingredients were dissolved in water to prepare a 1,000 ml solution. pH was adjusted to 7.0 to 7.3 by using a sodium hydroxide solution.

### (2): Production medium

L-asparagine (monohydrate) 4.0 g
Citric acid (monohydrate) 2.0 g
Magnesium sulfate (heptahydrate) 0.5 g
Potassium monohydrogenphosphate (anhydride) 0.5 g
Ammonium iron citrate 0.05 g
Glycerol 60 ml

The above ingredients were dissolved in water to prepare a 1,000 ml solution and sterilized with high pressure steam (121°C, 20 minutes). pH was adjusted to 7.0 to 7.3 by using a sodium hydroxide solution.

The physicochemical properties of the resulting Extract Z solution were as follows.
(1) Appearance:
   Pale yellow clear liquid
(2) pH:
   4.50 to 5.30
(3) Protein content:
   3.5 wt.% (as an amino acid) in a lyophilized product
(4) Nucleic acid content:
   0.1 wt.% in a lyophilized product
(5) Primary constituent monosaccharides of polysaccharide:
   Mannose 43.4 wt.%, arabinose 18.2 wt.%, and glucose 10.4 wt.% (hydrolyzed with 2N trifluoroacetic acid for two hours at 100°C, and then subjected to liquid chromatography using 2-cyanoacetamide fluorescent derivative (S. Honda, et al., Anal. Chem., 52, 1079 (1980)).

The Extract Z solution prepared by the method described in the Manufacturing Example described above can be appropriately diluted prior to use. In the following Examples, the Extract Z solution was diluted 1 to 50,000-fold and adjusted to a suitable concentration for use.

The manufacturers and catalogue numbers of the antibodies and staining reagents used in the following Examples are as follows.
CD80 (Miltenyi Biotec, catalogue number 130-102-372)
CD86 (Miltenyi Biotec, catalogue number 130-102-506)
CD11b (Miltenyi Biotec, catalogue number 130-113-811)
CD11c (Miltenyi Biotec, catalogue number 130-122-016)
CD45 (Miltenyi Biotec, catalogue number 130-119-130)
CD4 (Miltenyi Biotec, catalogue number 130-123-899)
CD8 (Life Technologies Corporation, catalogue number 25-0081-82)
TCRβ (BioLegend Incorporated, catalogue number 109220)
NK (anti-CD49b antibody; Miltenyi Biotec, catalogue number 130-102-258)
MHC class II (Miltenyi Biotec, catalogue number 130-123-785)
PD-L1 (anti-CD274 antibody; Life Technologies Corporation, catalogue number 12-5982-81)
PI (Propidium Iodide Solution; dead cell marker; Miltenyi Biotec, catalogue number 130-093-233)

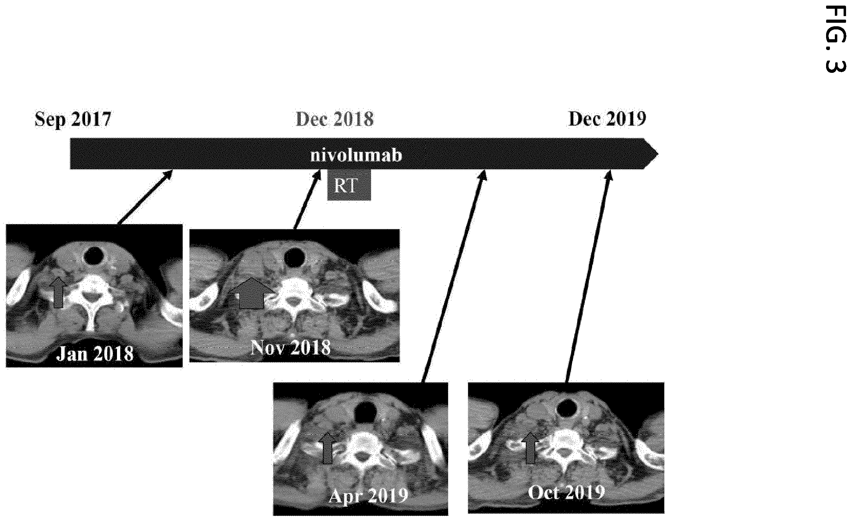

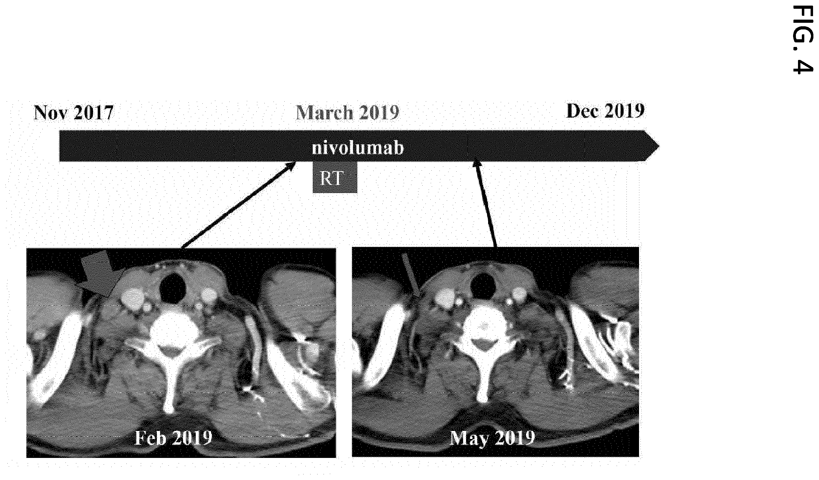

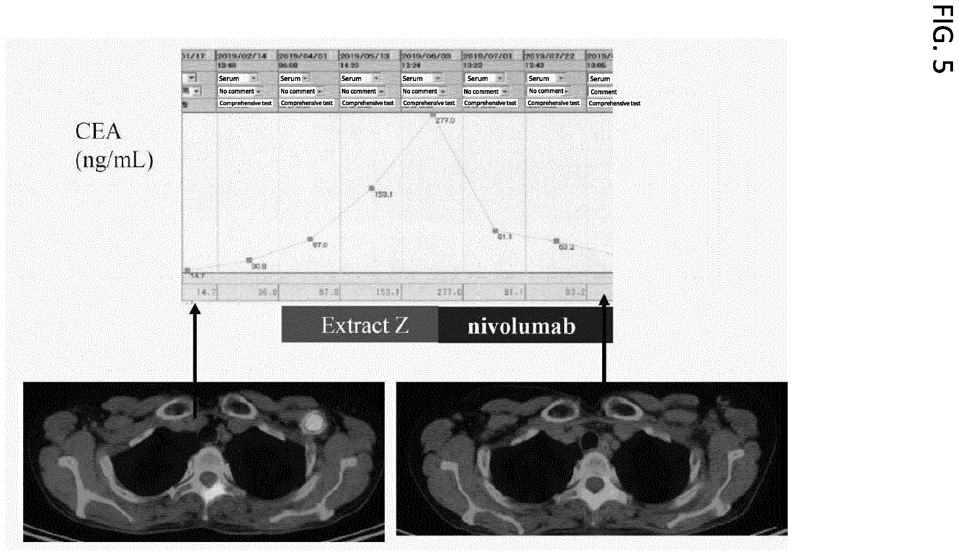

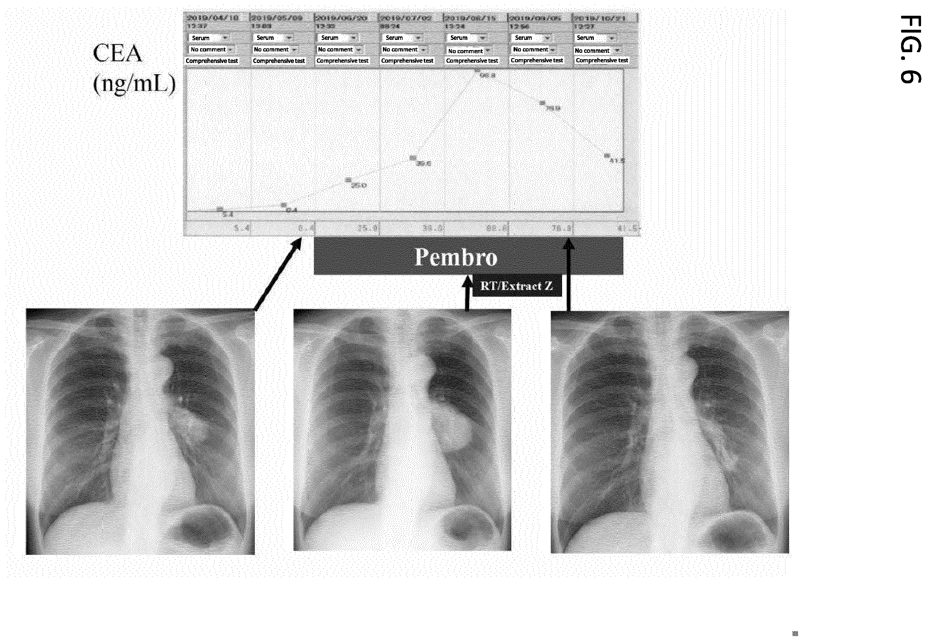

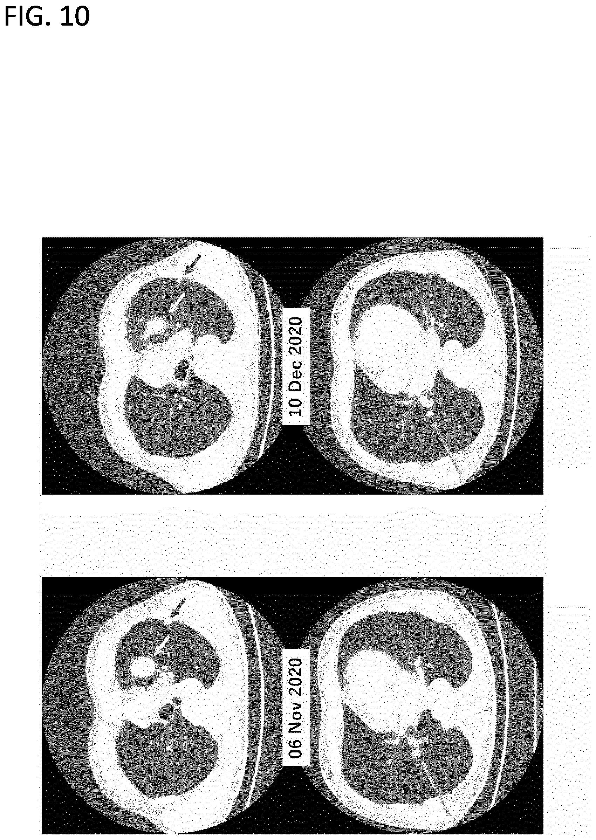

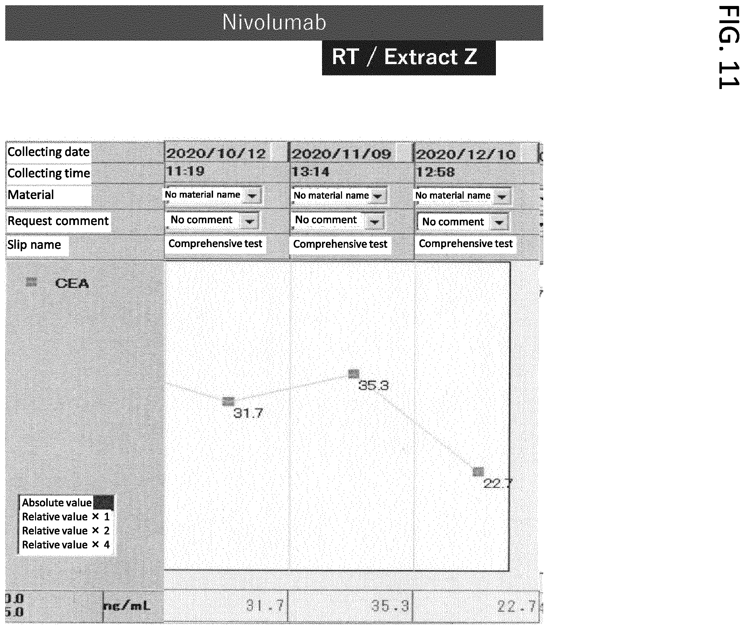

Examples of specific treatment for cancer patients are described below while referring to Figure 3 (Example 1: example 1 that is immune checkpoint inhibitor + radiation irradiation alone), Figure 4 (Example 2: example 2 that is immune checkpoint inhibitor + radiation irradiation alone), Figure 5 (Example 3: example of an immune checkpoint inhibitor after administration of Extract Z), Figure 6 (Example 4: example that is combined use of immune checkpoint inhibitor + administration of Extract Z + radiation irradiation), and Figures 10 and 11 (example that is combined use of immune checkpoint inhibitor + administration of Extract Z + radiation irradiation for a subject who received therapy with a tyrosine kinase inhibitor and therapy with an immune checkpoint inhibitor which were effective, but subsequently experienced progressive disease (PD)).

### (Example 1)

This Example presents a case of a 69-year-old male diagnosed with lung squamous cell carcinoma without a driver gene mutation, with clinical TNM classification: cTxN3M1b and at stage: IVb.

From September, 2017 to November, 2018, nivolumab, which is one type of anti-PD-1 antibody that is an immune checkpoint inhibitor, was administered via intravenous drip injection at 240 mg per dose at an interval of 2 weeks. However, swelling in the cervical lymph node to which cancer had been metastasized progressed as shown in the CT images of Figure 3. In December, 2018, the cervical lymph node was irradiated with a radiation of 30 Gy/10 fr. In addition, administration of nivolumab via intravenous drip injection at 240 mg per dose at an interval of 2 weeks has been continued from December, 2018 to the present (December, 2019). As a result, the swelling in the cervical lymph node was suppressed (April and October, 2019) and partially remitted, wherein the treatment has exerted the effect.

### (Example 2)

This Example presents a case of a 60-year-old male diagnosed with lung adenocarcinoma without a driver gene mutation, with recurrent TNM classification: rTxN3M1b and at stage: IVb.

From November, 2017 to February, 2019, nivolumab was administered via intravenous drip injection at 240 mg per dose at an interval of 2 weeks. However, swelling in the cervical lymph node to which cancer had been metastasized progressed as shown in the CT images of Figure 4. In March, 2019, the cervical lymph node was irradiated with a radiation of 30 Gy/10 fr. In addition, administration of nivolumab via intravenous drip injection at 240 mg per dose at an interval of 2 weeks has been continued from March, 2019 to the present (December, 2019). As a result, the swelling in the cervical lymph node was suppressed (May, 2019) and partially remitted, wherein the treatment has exerted the effect.

### (Example 3)

This Example presents a case of a 67-year-old female diagnosed with EGFR gene mutation positive (exon 19 deletion) lung adenocarcinoma, with clinical TNM classification: cT1bN3M1b and at stage: IVb.

From April 1, 2019, administration of Extract Z was started. Since the value of a tumor marker (CEA) increased from 57.0 to 277.0, administration of Extract Z was discontinued on June 3, 2019, and administration of nivolumab via intravenous drip injection at 240 mg per dose at an interval of 2 weeks was started. Thereafter, the value of the tumor marker (CEA) drastically decreased from 277.0 to 63.2.

As shown in the CT images of Figure 5, cancer tissue that had existed before the treatment was almost eliminated after the treatment. The synergistic action of administration of Extract Z and administration of nivolumab resulted in an excellent therapeutic effect.

### (Example 4)

This Example presents a case of a 55-year-old female diagnosed with EGFR gene mutation positive (exon 19 deletion) lung adenocarcinoma, with clinical TNM classification: cT3bN2M1c and at stage: IVb.

From June 20, 2019, pembrolizumab, which is one type of anti-PD-1 antibody that is an immune checkpoint inhibitor, started to be administered via intravenous drip injection at 200 mg per dose at an interval of 3 weeks. Since the value of a tumor marker (CEA) increased from 25.0 to 98.8, administration of Extract Z was started on August 15, 2019 while maintaining the administration of pembrolizumab. In doing so, radiation irradiation at 30 Gy/10 fr was performed in combination with the administration of Extract Z. Thereafter, the measurement value of the tumor marker (CEA) drastically decreased from 98.8 to 41.5.

As shown in the X-ray images of Figure 6, although the cancer tissue region once became larger on August 15 during the treatment (before administration of Extract Z), the region became much smaller after the treatment (administration of Extract Z + radiation irradiation) as compared to the region before the treatment. The synergistic action of administration of pembrolizumab and combined use of administration of Extract Z + radiation irradiation resulted in an excellent therapeutic effect.

### (Example 5: Application of immunoadjuvant/radiation therapy for directly activating a dendritic cell)

In this Example, application of an immunoadjuvant/radiation therapy for directly activating a dendritic cell was confirmed.

### (Material and method)

Immunoadjuvant = Extract Z
Radiation therapy

### (Example 6: Example of direct activation of dendritic cells by administration of an immunoadjuvant)

This Example shows direct activation of dendritic cells by administration of an immunoadjuvant.

1 mg/kg of saline or Extract Z is subcutaneously administered to the right inguinal region of a C3H/HeN mouse once daily (20 mice for each group). 1 × 10⁶ oral squamous cell carcinoma Sq-1979 cells are subcutaneously infused on the ventral side on day 29 after starting the administration of saline or Extract Z.

On day 36 after starting the administration of saline or Extract Z, tumor and lymph nodes are collected, the collected samples of 5 mice are pooled, and measurement is performed using a flow cytometer.

### (Antibody used)

Each antibody against CD80, CD86, CD11b, and CD11c

### (Result)

An increase in the amount of expression of CD80/86 on the surface of a dendritic cell was observed in the Extract Z administered group. This result suggests that administration of Extract Z activates a dendritic cell.

### (Example 7: Example of direct activation of a dendritic cell by administration of radiation therapy)

This Example shows direct activation of a dendritic cell by administration of radiation therapy.

Cancer cells are subcutaneously administered to mice to create mice with subcutaneous tumor. After subcutaneous transplantation of cancer cells, half the mice were subcutaneously and topically irradiated with a radiation. After the irradiation, subcutaneous tumor and lymph nodes are collected from the mice of the irradiation group and the mice of the non-irradiation group, and the amount of expression of CD80/86 on the surface of a dendritic cell is measured with a flow cytometer.

On day 36 after starting the irradiation (at the same timing for the non-irradiation group), tumor and lymph nodes are collected, the collected samples of 5 mice are pooled, and measurement is performed using a flow cytometer.

### (Antibody used)

Each antibody against CD80, CD86, CD11b, and CD11c

### (Example 8: Combined use of a plurality of techniques of directly activating a dendritic cell)

This Example shows combined use of a plurality of techniques of directly activating a dendritic cell.

Saline or Extract Z is administered to a group of patients with cervical cancer at a stage for which chemical radiation therapy is suitable. Administration of a platinum formulation (cisplatin or carboplatin) and radical radiation irradiation are performed at the same time as or at a different time from saline or Extract Z. Subsequently, immune parameters (CD11c, CD14, CD16, CD19, CD24, CD27, CD38, CD80, CD86, CD123, CD138, CCR5, CCR7, CXCR3, HLA-DR, CD3, CD4, CD8, CD45RA, CD56, CD69, CD159a, CTLA-4, NKp46, PD-1, IFNγ, TNFα, perforin, granzyme B, IL4, FoxP3, IL17A, Ki67, CXCL9, CXCL10, IL10, IL12p70), PFS (Progression-Free Survival), OS (Overall Survival), and ORR (Objective Response Rate) in the group of patients are measured.

### (Example 9: Combined use of an immune checkpoint inhibitor and an immunoadjuvant)

This Example shows combined use of an immune checkpoint inhibitor and an immunoadjuvant.

Saline or Extract Z is administered to a group of patients who have solid tumor, have no existing therapeutic choice, and are Tumor Mutational Burden^{high} and CD8⁺ T Cell invasion^{low}. An immune checkpoint inhibitor (e.g., pembrolizumab) is administered at the same time as or at a different time from saline or Extract Z. Subsequently, PFS (Progression-Free Survival), OS (Overall Survival), and ORR (Objective Response Rate) in the group of patients are measured.

### (Example 10: Another example of combined use of an immune checkpoint inhibitor and an immunoadjuvant)

This Example shows one example of combined use of an immune checkpoint inhibitor and an immunoadjuvant.

Extract Z is administered to a case with non-small cell lung cancer for which therapy with an immune checkpoint inhibitor alone was ineffective. Administration of an immune checkpoint inhibitor and palliative radiation therapy are performed at the same time as or at a different time from Extract Z. Subsequently, PFS (Progression-Free Survival), OS (Overall Survival), and ORR (Objective Response Rate) in the group of patients are measured.

### (Example 11: Combined use of an immune checkpoint inhibitor, an immunoadjuvant, and radiation therapy)

This Example shows an example of combined use of an immune checkpoint inhibitor, an immunoadjuvant, and radiation therapy.

Saline or Extract Z is administered to a group of patients who have non-small cell lung cancer, have no existing therapeutic choice, and EGFR mutation positive and EGFR-TKI intolerant/refractory. Administration of an immune checkpoint inhibitor and palliative radiation therapy are performed at the same time as or at a different time from saline or Extract Z. Subsequently, PFS (Progression-Free Survival), OS (Overall Survival), and ORR (Objective Response Rate) in the group of patients are measured.

### (Example 12: Combined use of an immune checkpoint inhibitor, an immunoadjuvant, radiation therapy, and a COX-2 inhibitor)

Extract Z is administered to a case with non-small cell lung cancer for which therapy with an immune checkpoint inhibitor alone was ineffective. Administration of an immune checkpoint inhibitor, palliative radiation therapy, and administration of a regulatory T cell inhibitor are performed at the same time as or at a different time from Extract Z. Subsequently, PFS (Progression-Free Survival), OS (Overall Survival), and ORR (Objective Response Rate) in the group of patients are measured.

### (Example 13: Example of enhancement of CXCL10 production by an immunoadjuvant)

This Example demonstrates enhancement of CXCL10 production by the agent of the present disclosure.

Bone marrow-derived cells were collected from a C3H/HeJ mouse (male), and the collected bone marrow-derived cells were cultured at 4 × 10⁶/plate for 6 days (in the presence of 20 ng/mL GM-CSF and 20 ng/mL IL-4). The bone marrow-derived cells after culture were collected, seeded at 2 × 10⁵/well, and stimulated with 0.4 µg/mL, 0.8 µg/mL, 1.6 µg/mL, and 3.2 µg/mL of Extract Z. The culture supernatant was collected after 6 hours from the stimulation, and the concentration of CXCL10 was measured by ELISA method.

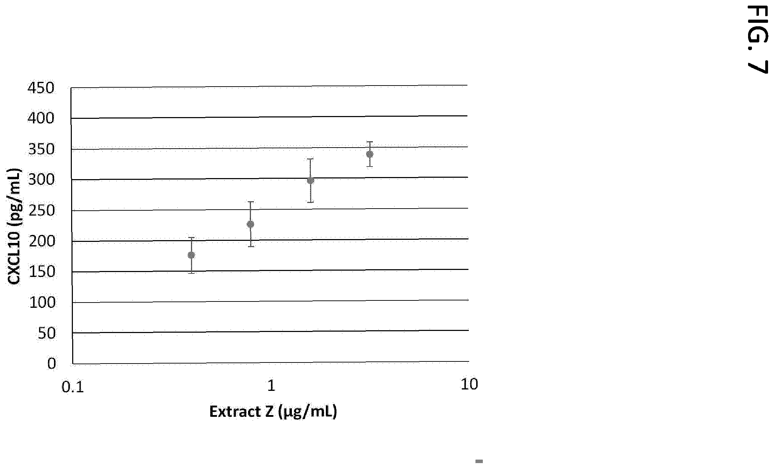

Figure 7 shows the result. An increase in the concentration of CXCL10 in the culture supernatant was observed as the concentration of Extract Z used for stimulation was increased. Thus, it was found that Extract Z has an action of enhancing CXCL10 production.

### (Example 14: Example of treatment of cancer or tumor that is CXCL10 positive by an immunoadjuvant)

### (CXCL10 positive)

An immunoadjuvant is administered to a model subcutaneously transplanted with cancer cells to confirm the antitumor effect or life-prolonging effect. Tumor is collected from a subcutaneously transplanted model in which the antitumor effect or life-prolonging effect was observed, and the amount of expression of the CXCL10 gene in the tumor is studied to confirm that the amount of expression is high.

### (CXCR3 positive)

An immunoadjuvant is administered to a model subcutaneously transplanted with cancer cells to confirm the antitumor effect or life-prolonging effect. Tumor is collected from a subcutaneously transplanted model in which the antitumor effect or life-prolonging effect was observed, and the CXCR3 positive cells in the tumor are studied by a flow cytometer or immunostaining to confirm that the positive cell ratio is high.

### (Example 15: Example of the effect of promoting infiltration of CD8⁺ T cells into tumor)

This Example shows the effect of the agent of the present disclosure to promote infiltration of CD8⁺ T cells into tumor.

1 mg/kg of saline or Extract Z was subcutaneously administered to the right inguinal region of a C3H/HeN mouse once daily (40 mice for each group). 1 × 10⁶ oral squamous cell carcinoma Sq-1979 cells were subcutaneously infused on the ventral side on day 29 after starting the administration of saline or Extract Z. Tumor was collected after 7 days from introduction of Sq-1979 cells, the samples of 5 mice were pooled, and measurement was performed using a flow cytometer.

### (Antibody and reagent used)

Antibody against CD45
Antibody against CD8
Antibody against TCRβ chain
PI (dead cell marker)

### (Result)

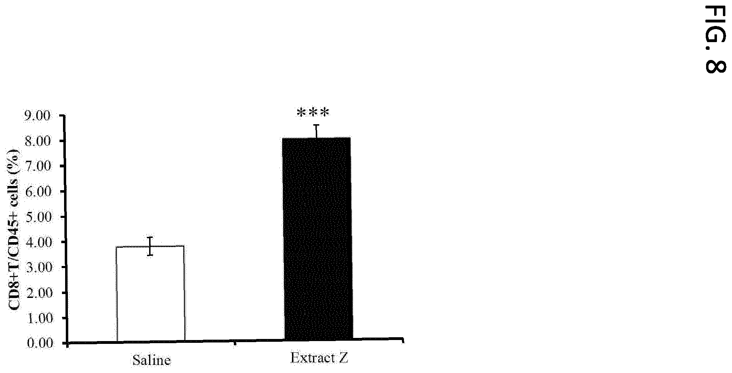

Figure 8 shows the result. Administration of Extract Z resulted in an increase in CD8⁺ T cells in tumor. Meanwhile, since the action of Extract Z is non-specific immunostimulatory, CD8⁺ T cells infiltrating into tumor include both cells having the antitumor effect and cells that express CTLA-4 and inhibit the antitumor effect.

### (Example 16: Example of treatment or prevention of Tumor Mutational Burden^{high} and CD8⁺ T cell^{low} inoperable or metastatic solid cancer for which there is no other therapeutic choice, wherein the treatment or prevention comprises an immunoadjuvant)

This Example shows demonstration of treatment or prevention of Tumor Mutational Burden^{high} and CD8⁺ T cell^{low} inoperable or metastatic solid cancer for which there is no other therapeutic choice, wherein the treatment or prevention comprises an immunoadjuvant.

Saline or Extract Z is administered to a group of patients who have solid cancer, have no existing therapeutic choice, and are Tumor Mutational Burden^{high} and CD8⁺ T Cell invasion^{low}. An immune checkpoint inhibitor (e.g., pembrolizumab) is administered at the same time as or at a different time from saline or Extract Z. Subsequently, PFS (Progression-Free Survival), OS (Overall Survival), and ORR (Objective Response Rate) in the group of patients are measured.

### (Example 17: Regimen treatment example)

This Example shows an example of a combined regimen of an immune checkpoint inhibitor, radiation therapy, and Extract Z.
(1) Immune checkpoint inhibitor: any of the following agents is used alone.
   (a) Intravenous drip injection of 240 mg of nivolumab for every 2 weeks in principle
   (b) Intravenous drip injection of pembrolizumab at 200 mg per dose for 30 minutes at an interval of 3 weeks, or intravenous drip injection of pembrolizumab at 400 mg per dose for 30 minutes at an interval of 6 weeks in principle
   (c) Intravenous drip injection of atezolizumab at 1200 mg per dose for 30 minutes at an interval of 3 weeks in principle
(2) Radiation therapy
   Palliative irradiation other than brain metastasis, wherein irradiation is performed at 2 Gy × 20 to 25 fractions or a dose equivalent dose: e.g., 3 Gy × 10 fractions. The irradiation method of palliative irradiation depends on the judgement of a radiation oncologist of each facility.
(3) Extract Z
   (a) Aspirating a liquid medicine into a 1 mL syringe and subcutaneously injecting a portion thereof (preferably about 0.05 mL) to the upper arm.
   (b) Twice a week
   (c) From the start of radiation therapy to the end of the radiation therapy (8 weeks at the maximum)
(4) Repeating radiation therapy and Extract Z
   After 9 months from the start of the therapy, radiation therapy and Extract Z may be repeated in another site.

### (Example 18: Example showing the ratio of tumor infiltrating cells as a result of administration of Extract Z)

This Example shows analysis of the ratio of tumor infiltrating cells as a result of administration of the agent of the present disclosure.

1 mg/kg of saline or Extract Z was repeatedly and subcutaneously administered to the right inguinal region of a C3H/HeN mouse once daily for 35 days (20 mice for each group). For Table 1, 1 × 10⁶ oral squamous cell carcinoma Sq-1979 cells were subcutaneously infused on the ventral side on day 29 after starting the administration of saline or Extract Z, and 200 µg/body of anti-PD-1 antibody was intraperitoneally infused on day 28, day 31, and day 34 after starting the administration of saline or Extract Z. For Table 2, 1 × 10⁶ oral squamous cell carcinoma Sq-1979 cells were subcutaneously infused on the ventral side on day 29 after starting the administration of saline or Extract Z, and 200 µg/body of anti-PD-1 antibody or Control antibody was intraperitoneally infused on day 28, day 31, and day 34 after starting the administration of saline or Extract Z.

On day 36 after starting the administration of saline or Extract Z, tumor and lymph nodes were collected, the collected samples of 5 mice were pooled, and measurement was performed using a flow cytometer.

### (Antibody and reagent used)

Antibodies against CD45, CD4, CD8, and TCR (T cell receptor)
Antibodies against NK, MHC class II, and PD-L1
PI (dead cell marker)

### (Result)

Table 1 and Table 2 show the results. While the percentage of CD8⁺ T cells in tumor infiltrating cells is 4.34% in the saline administered group, the percentage of CD8⁺ T cells is 10.43% in the Extract Z administered group (Table 1). In addition, while the group administered with an anti-PD-1 antibody alone shows 3.91%, the group administered with both Extract Z and an anti-PD-1 antibody shows 11.58% (Table 2). These results demonstrate that administration of Extract Z induces infiltration of CD8⁺ T cells into tumor.

### [Table 1]

**Table 1**

| | | Mean (cell ratio (%]) | SE |
|---|---|---|---|
| CD8+T | Saline | 4.34 | 0.07 |
| | Extract Z | 10.43 | 0.50 |
| CD4+T | Saline | 20.54 | 0.58 |
| | Extract Z | 22.46 | 1.05 |
| MHC class II | Saline | 6.71 | 0.53 |
| | Extract Z | 5.64 | 0.68 |
| NK | Saline | 39.79 | 1.35 |
| | Extract Z | 38.07 | 0.50 |
| PD-L1+/CD45+ | Saline | 68.62 | 1.36 |
| | Extract Z | 65.04 | 1.71 |
| PD-L1+/CD8+T | Saline | 96.79 | 0.18 |
| | Extract Z | 98.35 | 0.11 |

| | | | |
|---|---|---|---|
| n = 4, considering a pool of 5 mice as n = 1 **: p<0.01 **(**Aspin-Welch t-test) | | | |

### [Table 2]

**Table 2**

| Cell type | Sample | Cell ratio (%) | |
|---|---|---|---|
| | | Mean | SE |
| CD8+T/CD45+ | Saline + anti-PD-1 | 3.91 | 0.31 |
| | Extract Z + anti-PD-1 | 11.58 | 0.42 |
| CD4+T/CD45+ | Saline + anti-PD-1 | 29.93 | 2.98 |
| | Extract Z + anti-PD-1 | 26.58 | 2.72 |
| MHC class II+/CD45+ | Saline + anti-PD-1 | 4.81 | 1.08 |
| | Extract Z + anti-PD-1 | 4.36 | 0.19 |
| NK/CD45+ | Saline + anti-PD-1 | 27.02 | 1.21 |
| | Extract Z + anti-PD-1 | 30.88 | 1.87 |
| PD-L1+/CD45+ | Saline + anti-PD-1 | 67.88 | 2.19 |
| | Extract Z + anti-PD-1 | 66.42 | 3.52 |
| PD-L1+/CD8+T | Saline + anti-PD-1 | 99.02 | 0.28 |
| | Extract Z + anti-PD-1 | 99.30 | 0.15 |

| | | | |
|---|---|---|---|
| N = 4, considering a pool of 5 mice as N = 1 ^{∗∗∗} p<0.001 (student's t-test) | | | |

### (Example 19: Example showing the ratio of antigen-presenting cell markers in a lymph node as a result of administration of Extract Z)

This Example shows analysis of the ratio of antigen-presenting cell markers in a lymph node as a result of administration of the agent of the present disclosure.

1 mg/kg of saline or Extract Z was repeatedly and subcutaneously administered to the right inguinal region of a C3H/HeN mouse once daily for 35 days (20 mice for each group). For Table 3, 1 × 10⁶ oral squamous cell carcinoma Sq-1979 cells were subcutaneously infused on the ventral side on day 29 after starting the administration of saline or Extract Z, and 200 µg/body of anti-PD-1 antibody was intraperitoneally infused on day 28, day 31, and day 34 after starting the administration of saline or Extract Z. For Table 4, 1 × 10⁶ oral squamous cell carcinoma Sq-1979 cells were subcutaneously infused on the ventral side on day 29 after starting the administration of saline or Extract Z, and 200 µg/body of anti-PD-1 antibody or Control antibody was intraperitoneally infused on day 28, day 31, and day 34 after starting the administration of saline or Extract Z.

On day 36 after starting the administration of saline or Extract Z, tumor and lymph nodes were collected, the collected samples of 5 mice were pooled, and measurement was performed using a flow cytometer.

### (Antibody and reagent used)

Antibodies against CD45, CD80, CD86, CD11b, CD11c, MHC class II, and PD-L1
PI (dead cell marker)

### (Result)

Tables 3 and 4 show the results. While the percentage of CD80+/MHC class II cells in antigen-presenting cell markers in lymph nodes is 6.09% in the saline administered group, the percentage of CD80+/MHC class II cells is 8.38% in the Extract Z administered group. In addition, while the group administered with an anti-PD-1 antibody alone shows 6.08%, the group administered with both Extract Z and an anti-PD-1 antibody is 6.89% (Table 4). These results demonstrate that administration of Extract Z enhances CD80⁺ expression.

### [Table 3]

**Table 3**

| | | Mean (cell ratio [%]) | SE |
|---|---|---|---|
| CD80+/MHC class II | Saline | 6.09 | 0.12 |
| | Extract Z | 8,38 | 0.60 |
| CD86+/MHC class II | Saline | 28.44 | 1.01 |
| | Extract Z | 26.37 | 1.11 |
| CD11b+CD11c-/MHC class II | Saline | 0.85 | 0.03 |
| | Extract Z | 1.12 | 0.03 |
| CD11b+CD11c+/MHC class II | Saline | 1.28 | 0.06 |
| | Extract Z | 1.51 | 0.08 |
| CD11b-C D11c+/MHC class II | Saline | 13.03 | 0.44 |
| | Extract Z | 9.22 | 0.53 |

| | | | |
|---|---|---|---|
| n = 4, considering a pool of 5 mice as n = 1 *: p<0.05 (Aspin-Welch t-test) | | | |

### [Table 4]

**Table 4**

| Cell type | Sample | Cell ratio (%) | |
|---|---|---|---|
| | | Mean | SE |
| CD80+/MHC class II | Saline + anti-PD-1 | 6.08 | 0.27 |
| | Extract Z + anti-PD-1 | 6.89 | 0.22 |
| CD86+/MHC class II | Saline + anti-PD-1 | 25.90 | 0.82 |
| | Extract Z + anti-PD-1 | 24<65 | 1.78 |
| CD11b+CD11c-/ MHC class II | Saline + anti-PD-1 | 1.18 | 0.11 |
| | Extract Z + anti-PD-1 | 1.41 | 0.06 |
| CD11b+CD11c+/ MHC class II | Saline + anti-PD-1 | 0.84 | 0.06 |
| | Extract Z + anti-PD-1 | 0.86 | 0.04 |
| CD11b-CD11c+/ MHC class II | Saline + anti-PD-1 | 10.51 | 0.50 |
| | Extract Z + anti-PD-1 | 9.76 | 0.61 |

| | | | |
|---|---|---|---|
| N = 4, considering a pool of 5 mice as N = 1 | | | |

### (Example 20: Example showing PD-L1 expression in cultured Sq-1979 cells)

This Example shows analysis of PD-L1 expression in cultured Sq-1979 cells by the agent of the present disclosure.

Cultured oral squamous cell carcinoma Sq-1979 cells were collected and stained with an anti-PD-Ll antibody and a Control antibody, and measurement was performed using a flow cytometer.

### (Result)

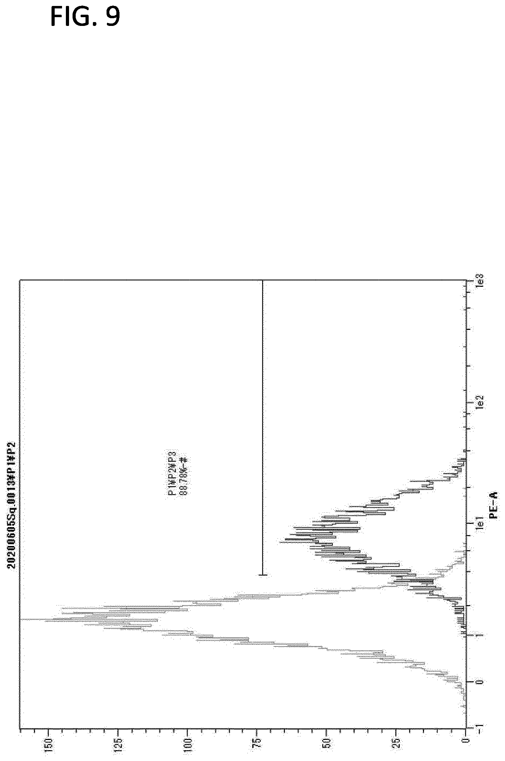

Figure 9 shows the result. When stained with an anti-PD-L1 antibody, 88.78% cells were positive, whereas when stained with a Control antibody, 1.57% cells were positive. Thus, it was demonstrated that Sq-1979 cells are PD-L1 positive cells.

### (Example 21: The effect of combined use of Extract Z and an immune checkpoint inhibitor)

In this Example, Extract Z and an anti-CTLA-4 antibody are administered to a mouse tumor model to confirm that the antitumor effect or life-prolonging effect synergistically increases.

A splenocyte or lymph node is collected from a mouse, and an immune cell is isolated and Extract Z and an anti-CTLA-4 antibody are added thereto. Extract Z exhibits a IFN-γ production action (T cell activation action) by being added in vitro. Thus, it is confirmed whether the IFN-γ production synergistically increases by combined use with a CTLA-4 antibody.

### (Example 22: Example showing that a CD8⁺ T cell infiltrating into tumor expresses CTLA-4⁺ by administration of Extract Z)

This Example confirms an increase in CTLA-4 expression in a tumor infiltrating CD8⁺ T cell which is found as a result of administration of Extract Z.

It is confirmed that administration of Extract Z not only promotes infiltration of overall CD8⁺ T cells but also promotes infiltration of CTLA-4⁺ CD8⁺ T cells that inhibits antitumor effect.

### (Example 23: Example of treatment with Extract Z, an immune checkpoint inhibitor, and radiation irradiation)

In this Example, the therapeutic effect of combined use of Extract Z, an immune checkpoint inhibitor, and radiation irradiation was confirmed.

This Example presents a case of a 57-year-old male diagnosed with EGFR gene mutation positive (exon 19 deletion) lung adenocarcinoma, with clinical TNM classification: cT2aN2M1c and at stage: IVb.

After administration of afatinib was started from October, 2015, recurrence having a T790M mutation was observed in 2016. Thus, administration of osimertinib was started from December, 2016. Since progression was observed in June, 2017, chemotherapy with cisplatin + pemetrexed was performed, followed by performing treatment with erlotinib + bevacizumab. However, progression was observed again. Therefore, administration of nivolumab via intravenous drip injection at 240 mg per dose at an interval of 2 weeks was started from September, 2017. As a result, primary foci were almost eliminated and metastasis to the lymph nodes was eliminated, but progression of primary tumor, multiple metastases in the lung, and metastasis to the right adrenal gland were observed on November 6, 2020.

From November 17, 2020 to November 28, 2020, radiation irradiation at 30 Gy/10 fr and four times of administration of Extract Z were performed on the right adrenal gland metastatic site while continuing the administration of nivolumab.

As a result, the measurement value of a tumor marker (CEA) decreased from 35.3 to 22.7. Furthermore, it was found that the primary foci slightly reduced and some of the metastatic foci in the lung obviously reduced after the treatment (administration of Extract Z + radiation irradiation) as shown in the CT images of Figure 10.

It is considered that the synergistic effect of administration of nivolumab and combined use of administration of Extract Z + radiation irradiation resulted in an abscopal effect. It was also demonstrated that an excellent therapeutic effect is attained in a later therapy-line patient, in which progression is observed although all standard therapies were performed.

### (Note)

As described above, the present disclosure is exemplified by the use of its preferred embodiments. However, it is understood that the scope of the present disclosure should be interpreted solely based on the Claims. It is also understood that any patent, any patent application, and any references cited herein should be incorporated herein by reference in the same manner as the contents are specifically described herein. The present application claims priority to Japanese Patent Application No. 2019-238657 filed on December 27, 2019 with the Japan Patent Office, and Japanese Patent Application No. 2020-171493 filed on October 9, 2020 with the Japan Patent Office. It is understood that the entire content thereof is incorporated herein by reference.

### [Industrial Applicability]

The present disclosure provides a method of preventing and treating a disease such as cancer based on a conventionally unavailable mechanism.

## Claims

1. A combination for use in treatment, prevention, or prevention of recurrence of cancer or tumor, comprising:
a) an immune checkpoint inhibitor; and
b) a direct dendritic cell activating agent or means,
**characterized in that** a) and b) are administered at different times or at the same time.

2. A composition or medical device for use in treatment, prevention, or prevention of recurrence of cancer or tumor of a subject, comprising a direct dendritic cell activating agent or means, **characterized in that** the composition or medical device is administered or used in combination with an immune checkpoint inhibitor.

3. A composition for use in treatment, prevention, or prevention of recurrence of cancer or tumor of a subject, comprising an immune checkpoint inhibitor, **characterized in that** the composition is administered in combination with a direct dendritic cell activating agent or means.

4. The combination, composition, or medical device of any one of claims 1 to 3, wherein the direct dendritic cell activating agent or means comprises at least one selected from the group consisting of a radiation therapy providing means and an immunoadjuvant.

5. The combination, composition, or medical device of any one of claims 1 to 3, wherein the direct dendritic cell activating agent or means comprises a radiation therapy providing means and an immunoadjuvant.

6. The combination, composition, or medical device of any one of claims 1 to 5, wherein the immune checkpoint inhibitor comprises an inhibitor against at least one factor selected from the group consisting of PD-1, PD-L1, and CTLA-4.

7. The combination, composition, or medical device of any one of claims 1 to 5, wherein the immune checkpoint inhibitor comprises at least one agent selected from the group consisting of a PD-1 inhibitor, a PD-L1 inhibitor, and a CTLA-4 inhibitor.

8. The combination, composition, or medical device of any one of claims 1 to 7, wherein the cancer or tumor expresses an immune checkpoint factor.

9. The combination, composition, or medical device of any one of claims 1 to 8, wherein the cancer expresses PD-L1.

10. The combination, composition, or medical device of any one of claims 1 to 9, wherein the immune checkpoint inhibitor comprises at least one selected from the group consisting of nivolumab, pembrolizumab, and atezolizumab.

11. The combination, composition, or medical device of any one of claims 1 to 10, wherein the cancer is lung cancer.

12. The combination, composition, or medical device of any one of claims 1 to 11, wherein the cancer or tumor has an EGFR gene mutation.

13. The combination, composition, or medical device of any one of claims 1 to 12, wherein the treatment, prevention, or prevention of recurrence is performed on a subject who has received therapy with a tyrosine kinase inhibitor.

14. The combination, composition, or medical device of any one of claims 1 to 13, wherein the treatment, prevention, or prevention of recurrence is performed on a subject who has not received therapy with an immune checkpoint inhibitor.

15. The combination, composition, or medical device of any one of claims 1 to 14, wherein the treatment, prevention, or prevention of recurrence is performed on a subject for whom therapy with an immune checkpoint inhibitor was determined to be ineffective.

16. The combination, composition, or medical device of any one of claims 1 to 15, wherein the treatment, prevention, or prevention of recurrence is performed on a subject who received therapy with an immune checkpoint inhibitor which was effective, but subsequently experienced progressive disease (PD).

17. The combination, composition, or medical device of any one of claims 1 to 15, wherein the treatment, prevention, or prevention of recurrence is performed on a subject who received therapy with a tyrosine kinase inhibitor and therapy with an immune checkpoint inhibitor which were effective, but subsequently experienced progressive disease (PD) .

18. The combination, composition, or medical device of any one of claims 1 to 17, wherein the subject is CXCL10 positive in the subject's tumor.

19. The combination, composition, or medical device of any one of claims 1 to 18, wherein the subject is a CD8⁺ T cell^{low} subject.

20. A composition for use in directly activating a dendritic cell, comprising an immunoadjuvant.

21. The composition of claim 20, wherein the immunoadjuvant comprises a human *Mycobacterium tuberculosis* hot water extract or a portion thereof.

22. The composition of claim 20 or 21, wherein the activation is determined by measuring an increase in an amount of expression of CD80/86 on a surface of a dendritic cell in the presence of the immunoadjuvant compared to in the absence of the immunoadjuvant.

23. A combination for use in treatment, prevention, or prevention of recurrence of cancer or tumor, comprising:
a) an immune checkpoint inhibitor; and
b) an immunoadjuvant,
**characterized in that** a) and b) are administered at different times or at the same time.

24. A composition for use in treatment, prevention, or prevention of recurrence of cancer or tumor of a subject, comprising an immunoadjuvant, **characterized in that** the composition is administered in combination with an immune checkpoint inhibitor.

25. A composition for use in treatment, prevention, or prevention of recurrence of cancer or tumor of a subject, comprising an immune checkpoint inhibitor, **characterized in that** the composition is administered in combination with an immunoadjuvant.

26. The combination or composition of any one of claims 23 to 25, wherein the immunoadjuvant comprises a human *Mycobacterium tuberculosis* hot water extract or a portion thereof.

27. The composition of any one of claims 23 to 26, wherein the immune checkpoint inhibitor comprises an inhibitor against at least one factor selected from the group consisting of PD-1, PD-L1, and CTLA-4.

28. The combination or composition of any one of claims 23 to 27, wherein the immune checkpoint inhibitor comprises at least one agent selected from the group consisting of a PD-1 inhibitor, a PD-L1 inhibitor, and a CTLA-4 inhibitor.

29. A combination, or a composition or medical device for use in activating a dendritic cell, comprising the composition of any one of claims 23 to 28 and a radiation therapy providing means.

30. The combination, composition, or medical device of claim 29, wherein the radiation therapy providing means comprises at least one selected from the group consisting of a radiosensitizer, a radiation irradiating device, and a radioactive substance.

31. The combination, composition, or medical device of claim 29 or 30, **characterized in that** the radiation therapy providing means is configured to provide palliative irradiation.

32. The combination, composition, or medical device of any one of claims 29 to 31, **characterized in that** the radiation therapy providing means is configured to irradiate to have an abscopal effect.

33. The combination, composition, or medical device of any one of claims 29 to 32, **characterized in that** the radiation therapy providing means is configured to irradiate a measurable lesion that is present other than a lesion that is a target.

34. The combination, composition, or medical device of any one of claims 23 to 33, further comprising a regulatory T cell inhibitor.

35. The combination, composition, or medical device of claim 34, wherein the regulatory T cell inhibitor comprises a COX-2 inhibitor.

36. The combination, composition, or medical device of claim 35, wherein the COX-2 inhibitor is selected from the group consisting of celecoxib, etodolac, meloxicam, nabumetone, zaltoprofen, and lornoxicam.

37. A composition or medical device for use in activating a dendritic cell, comprising a radiation therapy providing means.

38. The composition or medical device of claim 37, wherein the radiation therapy providing means comprises at least one selected from the group consisting of a radiosensitizer, a radiation irradiating device, and a radioactive substance.

39. The composition or medical device of claim 37 or 38, wherein the activation is determined by measuring an increase in an amount of expression of CD80/86 on a surface of a dendritic cell in the presence of the immunoadjuvant compared to in the absence of the immunoadjuvant.

40. The composition or medical device of any one of claims 37 to 39, **characterized in that** the radiation therapy providing means is configured to provide palliative irradiation.

41. The composition or medical device of any one of claims 37 to 40, **characterized in that** the radiation therapy providing means is configured to irradiate to have an abscopal effect.

42. The composition or medical device of any one of claims 37 to 41, **characterized in that** the radiation therapy providing means is configured to irradiate a measurable lesion that is present other than a lesion that is a target.

43. A program utilized for cancer treatment or prevention of a patient, **characterized by** causing a computer to execute:
a step of performing either one or both of first therapy with an immune checkpoint inhibitor and/or a dendritic cell activating agent or means on the patient; and
a step of identifying an optimal therapy program based on a reaction of the patient.

44. The program of claim 43, **characterized in that** the reaction of the patient is a predetermined change in a medical image of the patient and a measurement value of a tumor marker in the patient.

45. A medicament for use in prevention or treatment of cancer in a patient, the medicament comprising a combination of an immune checkpoint and a dendritic cell activating agent or means, **characterized in that** dosage and administration are identified based on information on an optimization identifying marker of the combination obtained from the patient, and a therapeutically or prophylactically effective combination of the immune checkpoint and the dendritic cell activating agent or means is administered based on the identified dosage and administration.

46. A medicament or device for use in treatment, prevention, or prevention of recurrence of cancer, cancer, or tumor of a subject with a specific regimen,
the medicament comprising a combination of an immune checkpoint and a dendritic cell activating agent or means, wherein the regimen comprises:
a) a step of administering an immune checkpoint inhibitor; and
b) a step of administering a dendritic cell activating agent.

47. The medicament or device of claim 46, wherein the subject is a subject who has not received therapy with an immune checkpoint inhibitor or a subject for whom therapy with an immune checkpoint inhibitor alone is ineffective.

48. The medicament or device of any one of claim 46 or 47, wherein, in the specific regimen, the immune checkpoint inhibitor:
i) is nivolumab and administered at 240 mg every 2 weeks;
ii) is pembrolizumab and administered at 200 mg at an interval of 3 weeks or at 400 mg at an interval of 6 weeks;
or
iii) is atezolizumab and administered at 1200 mg at an interval of 3 weeks.

49. The medicament or device of any one of claims 46 to 48, wherein the specific regimen further comprises:
c) a step of performing radiation therapy.

50. The medicament or device of claim 49, **characterized in that** the radiation therapy is palliative irradiation other than brain metastasis, and irradiation is performed 10 fractions at 3 Gy or 20 to 25 fractions at 2 Gy.

51. The medicament or device of any one of claims 46 to 50, wherein the dendritic cell activating agent or means comprises Extract Z that is administered by aspirating a liquid medicine into a 1 mL syringe and subcutaneously injecting 0.05 mL thereof twice a week.
